# EUROPEAN PATENT APPLICATION

(11) **EP 2 161 272 A1**
(43) Date of publication of application: **10.03.2010**
(21) Application number: 08163723.3
(22) Date of filing: 05.09.2008
(51) Int. Cl.: C07D 471/04, C09K 11/06, H05B 33/14, H01L 51/00

(54) **Phenanthrolines**

(71) Applicant: BASF SE, 67056 Ludwigshafen (DE)
(72) Inventor: Nesvadba, Peter, 1723 Marly (CH); Bugnon Folger, Lucienne, 4148 Pfeffingen (CH); Ricci, Andrea, 4052 Basel (CH); Moegle, Gilbert, 68510 Magstatt-le-Bas (CH)

(57) **Abstract**

The present invention relates to phenanthroline compounds and to electroluminescent (EL) devices comprising the phenanthroline compounds. The phenanthroline compounds may function as electron transport layers, as hole blocking layers or as hosts for phosphorescent emitter materials to provide electroluminescent devices with improved efficiency, lifetime, operational stability and better spectral characteristics.

## Description

The present invention relates to novel phenanthroline compounds and to electroluminescent (EL) devices comprising the novel phenanthroline compounds. The novel phenanthroline compounds may function as electron transport layers, as hole blocking layers or as hosts for fluorescent or phosphorescent emitter materials to provide electroluminescent devices with improved efficiency, lifetime, operational stability and better spectral characteristics.

Organic light emitting devices (OLED's) have attracted huge amount of research in Material Sciences during the last two decades. This development is summarized in extensive literature, for example in the book "Organic Light Emitting Devices", K. Müllen and U. Scherff, Ed., Wiley-VCH 2006.

In principle, OLED's include several organic layers in which at least one of the layers includes an organic material that can be made to electroluminescence by applying voltage across the device. For example one possible architecture of an OLED revealed in EP 1 097 980/A2 consists of a support, hole injecting electrode (anode), hole injecting layer, hole transporting layer, light emitting layer, hole blocking layer, electron transporting layer, electron injecting layer and electron injecting electrode (cathode). Different other OLED designs are known and described in the literature, for example in the below cited patent literature.

Amongst different materials suitable for manufacturing of OLED's layers are derivatives of 1,10-phenanthroline. Their use in OLED's has been disclosed several times in the past, for example in:

WO 2004/026870/A1, WO 2004/005288/ A2, WO 2004/006352/ A2, JP 2007/108720, JP 2002/100482, JP 2002/100479, EP 1 097981/A2, EP 1 097 980/A2, EP 0 818 943/A2, EP 0 564 224/A2, US 2007/0085106/A1, US 2007/0037983/A1, US 2007/7 186 469/B2, EP 1 786 050/A1, US 2007/0122657/A1, US 2004/0076853/A1, JP 2008/135498/A, DE 102007012794/B3.

Additionally metal complexes of phenanthroline compounds have also been disclosed as luminiscent materials for OLEDS, e.g.

WO 2005/056712/A1 teaches the use of PT(II) complexes and WO 00/64996 discloses lanthanide complexes as luminescent materials in OLEDS.

The charge carrier transportability by the phenanthroline compounds can be controlled by varying the type of substitution on the 1,10-phenanthroline skeleton. Yet another possibility for improvement of the carrier mobility and, hence, conductivity of the phenanthroline containing layers of the OLED device is their doping with preferably but not limited to electron donating (n-doping) compounds. This approach is disclosed for example in EP 1 786 850 /A1 or WO 2005/086251/A2 or DE 102007012794/B3 and literature cited therein.

Thus, phenanthroline compounds are known and the efficiency of some of them as building blocks for OLEDS has been demonstrated.

However, materials with still better properties are needed. Particularly desired properties are high melting points and high glass transition temperatures, as well as thermal, chemical and electrochemical stability. These properties positively influence the stability and life time of the OLED device in which they are used. Moreover, the compounds should be sublimable and thus depositable as amorphous films via vacuum deposition process.

Another important property is the right level of the highest occupied molecular orbital (HOMO) and lowest unoccupied molecular orbital (LUMO) in order to assure their energetic compatibility with the neighboring layers.

Moreover, there remains a need for EL devices comprising new electron transport layer materials, new blocking layer materials and new host materials, particularly those that afford improved EL device efficiency, lifetime, operational stability and better spectral characteristics.

These highly desired improvements have been achieved with the novel phenanthroline compounds according to the present invention.

An embodiment of the present invention is a compound of formula (I) or (II) 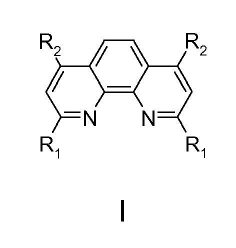 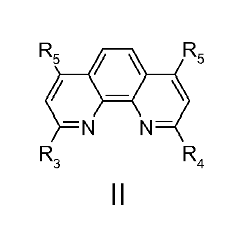 wherein
in formula (I) R₁ and R₂ are independently substituted or unsubstituted C₆-C₂₅ aryl,
with the proviso that,
if R₁ is unsubstituted phenyl, R₂ is not unsubstituted phenyl, and
if R₁ is aryl other than unsubstituted phenyl, R₂ is not unsubstituted phenyl, substituted or
unsubstituted 3-perylenyl ( 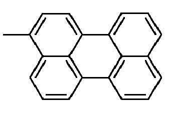 ), 2-fluorenyl ( 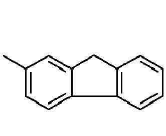 ) or 8-fluoranthenyl ( 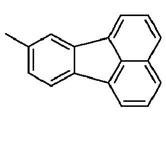 ), and
in formula (II) R₃ is C₃-C₂₅ heteroaryl, R₄ is H, C₁- C₁₈ alkyl, C₄- C₁₂ cycloalkyl, C₂-C₁₈alkenyl, C₂-C₁₈alkinyl, C₆-C₂₅ aryl, or C₃-C₂₅ heteroaryl, and R₅ is C₅- C₁₂ cycloalkyl, C₆-C₂₅ aryl, or C₃-C₂₅ heteroaryl, wherein R₃ to R₅ may optionally be substituted.

For instance for the denotations R₁ to R₅ occurring more than once in a molecule, each of this denotation is the same, of course, independently of the other denotations, i.e. R₁ is the same, independently thereof R₂ is the same, etc.

The optional substituents on R₁ to R₅ independently are one or more heteroatomgroups, for instance carbazyl, morpholyl, piperidyl, piperazyl, pyridyl, N-C₁-C₁₈alkylpiperazyl, halogen, - COOR₁₀,-CON(R₇)(R₉), -OR₁₀, -OC(O)R₇, -OC(O)OR₁₀, -OC(O)N(R₇)(R₉), -NR₇C(O)R₉, -CN, -O-CN, -NCO, -N₃, -NR₇C(O)N(R₉)(R₁₁), -N(R₇)(R₉), -NR₇COOR₁₀, -SR₁₀, -S(=O)R₇ or - S(=O)₂R₇ or combinations thereof; or one or more groups of C₁-C₁₈alkyl, C₂-C₁₈alkenyl, C₂-C₁₈alkinyl, C₃-C₁₂cycloalkyl, C₅-C₁₂cycloalkenyl or perfluorinated C₁-C₁₈alkyl.

R₇, R₉ and R₁₁ are independently H, C₁-C₁₈alkyl, C₂-C₁₈alkenyl, C₂-C₁₈alkynyl, C₇-C₁₅aralkyl, C₈-C₁₅aralkenyl, C₈-C₁₅aralkynyl, C₃-C₁₂cycloalkyl, C₆-C₂₀aryl or C₃-C₂₀heteroaryl.

R₁₀ is NH₄, Li, Na, K or is as defined for R₇.

In the definitions according to the invention in general and for the optional substituents of R₁ to R₅ in particular the following applies.

The term alkyl comprises within the given limits of carbon atoms, for example methyl, ethyl, propyl, isopropyl, n-butyl, sec-butyl, isobutyl, tert-butyl, 2-ethylbutyl, n-pentyl, isopentyl, 1-methylpentyl, 1,3-dimethylbutyl, n-hexyl, 1-methylhexyl, n-heptyl, 2-methylheptyl, 1,1,3,3-tetramethylbutyl, 1-methylheptyl, 3-methylheptyl, n-octyl, 2-ethylhexyl, 1,1,3-trimethylhexyl, 1,1,3,3-tetramethylpentyl, nonyl, decyl, undecyl, 1-methylundecyl or dodecyl.

Examples of alkenyl are within the given limits of carbon atoms vinyl, allyl, 1-methylethenyl, and the branched and unbranched isomers of butenyl, pentenyl, hexenyl, heptenyl, octenyl, nonenyl, decenyl, undecenyl and dodecenyl. The term alkenyl also comprises residues with more than one double bond that may be conjugated or non-conjugated, for example may comprise one double bond.

Examples of alkynyl are within the given limits of carbon atoms ethynyl, propargyl, 1-methylethynyl, and the branched and unbranched isomers of butynyl, pentynyl, hexynyl, heptynyl, octynyl, nonynyl, decynyl, undecynyl and dodecynyl, especially propargyl. The term alkynyl also comprises residues with more than one triple bond and residues with a triple bond and a double bond, all of which may be conjugated or non-conjugated. For instance, alkynyl comprises one triple bond.

Some examples of cycloalkyl are cyclopropyl, cyclobutyl, cyclopentyl, cyclohexyl, cycloheptyl, cyclooctyl, methylcyclopentyl, dimethylcyclopentyl, methylcyclohexyl and dimethylcyclohexyl, for instance cyclohexyl.

Some examples of cycloalkenyl are cyclopentenyl, cyclohexenyl, methylcyclopentenyl, dimethylcyclopentenyl and methylcyclohexenyl. Cycloalkenyl may comprise more than one double bond that may be conjugated or non-conjugated, for example may comprise one double bond.

Aryl is for example phenyl, perylenyl, fluorenyl, fluoranthenyl, naphthyl, phenantrenyl, anthryl, pyrenyl, biphenyl, benzoanthryl and dibenzoanthryl.

Heteroaryl comprises for instance the heteroatoms S, N and/or O (e.g. comprises 1-5, especially 1-3, in particular 1 or 2 such as 1 heteroatom), is a heterocyclic aromatic group and is for instance carbazyl, pyridyl, quinolyl, isoquinolyl, pyrryl, indolyl, isoindolyl, furyl, benzofuryl, dibenzofuryl, thiophenyl, benzothiophenyl, phenothiazinyl, benzoimidazyl, imidazyl, oxazyl, thiazyl, triazyl, pyridazyl, pyrimidyl, pyrazyl, puryl, pteridyl, acridinyl or phenazyl.

Aralkyl is for instance benzyl or α,α-dimethylbenzyl.

Aralkenyl is for example phenylethenyl.

Aralkynyl is for instance phenylethynyl.

The term halogen may comprise fluorine, chlorine, bromine and iodine, for example fluorine, chlorine or bromine, especially fluorine.

Perfluorinated alkyl, especially perfluorinated C₁-C₄alkyl, is for example -CF₃, -CF₂CF₃, - CF₂CF₂CF₃, -CF(CF₃)₂, -(CF₂)₃CF₃, or -C(CF₃)₃, in particular -CF₃.

The compounds of the present invention can be made according to the following general synthetic scheme in analogy to the published synthesis of 4,7-diphenyl-1,10-phenanthroline (J.Org. Chem. 1541 (1951)) or other 4,7-disubstituted-1,10-phenanthrolines (J.Org. Chem. 1641 (1962)).

Thus, 2-nitroaniline is subjected to a Skraup reaction with the readily available substituted 3-chloro-propiophenone R-COCH₂CH₂Cl to afford the corresponding 8-nitroquinoline which is reduced into the 8-aminoquinoline. Repeated Skraup reaction of the latter affords the intermediate 4,7-disubstituted 1,10-phenanthroline

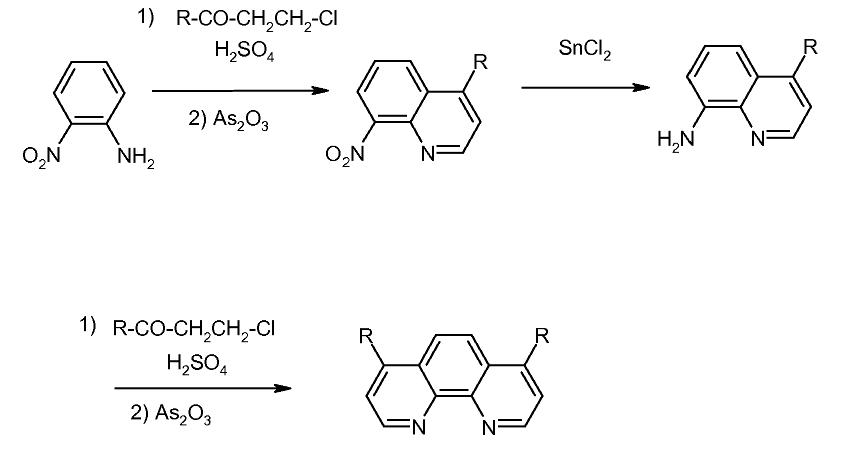

A more straightworward synthesis of the intermediate 4,7-disubstituted 1,10-phenanthrolines is described in the Czech Patent (CS 150747, 1,10-Phenanthroline derivatives (1973)) which uses the condensation of the same 3-chloro-propiophenone R-COCH₂CH₂Cl with 1,2-diaminobenzene under concomitant oxidation with chloranil:

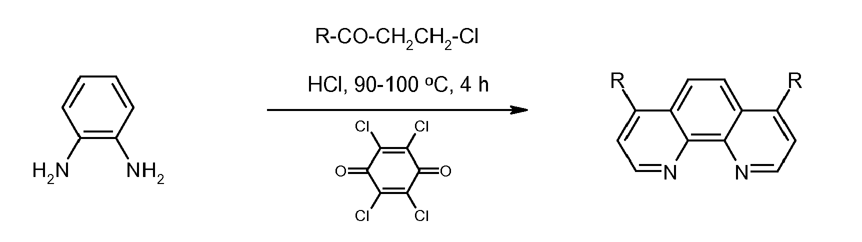

This method and its variations is preferred for the preparation of compounds of the present invention.

The starting 2-chloroethylketones R-COCH₂CH₂Cl are readily available via different methods. Examples are:
1) Friedel-Crafts acylation of the corresponding aromatic compounds with 3-chloropropionyl chloride, in analogy to the described (Journal of the American Chemical Society (1932), 54 736-48) synthesis of phenyl-2-chloroethyl ketone: 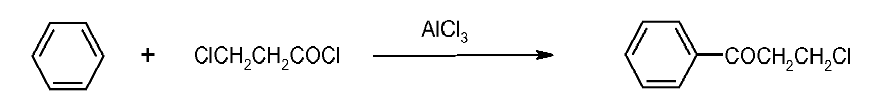
2) Addition of acyl chlorides to ethylene, in analogy to the described (Bioorganic & Medicinal Chemistry Letters (2005), 15(3), 589-593) synthesis of phenyl-2-chloroethyl ketone:

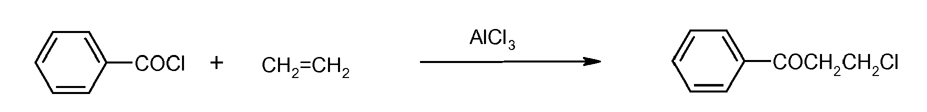

The starting aromatic compounds or acyl chlorides for the synthesis of the inventive compounds are commercial or readily available via standard methods of organic synthesis which do not need further discussion here.

The transformation of the intermediate 4,7-disubstituted 1,10-phenanthrolines into the compounds (I) or (II) can be performed via addition of a suitable organometallic, preferably organolithium compound Rₐ-Li followed by dehydrogenation, for example with MnO₂. Depending on the amount of the organolithium compound used, mono- (2-) or di- (2,9-) substituted-4,7-disubstituted-1,10-phenanthrolines are obtained.

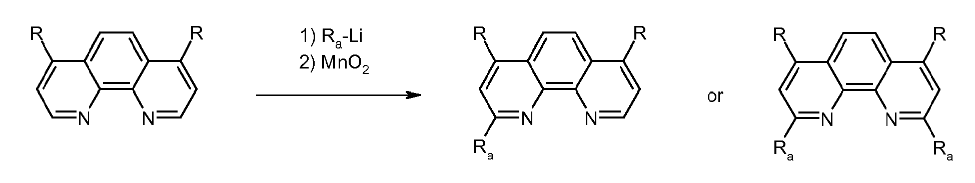

For example, EP 1097980/A2 and US 7 186 469/B2 describe the preparation of a variety of 2,4,7,9-tetrasubstituted phenanthrolines via addition of organolithium reagents to 4,7-diphenylphenanthroline and the use of such compounds as electron transport and hole blocking materials in OLED's.

Similarly, WO 2004/026870/A1 teaches the preparation of novel phenanthroline compounds via reaction of fluorenyl-, fluoranthenyl-, perylenyl- or carbazolyl organometallic compounds with a suitable phenanthroline precursor.

The preparation of asymmetric 2-substituted 4,7-diphenylphenanthrolines using the addition of one equivalent of an organolithium compound to 4,7-diphenylphenanthroline and the use of such compounds in OLED's is described in US 2007/0037983/ A1.

The addition of one equivalent of 2-pyridyllithium to 4,7-dimethylphenanthroline is described in Inorg. Chem. (2002), 41(24), 6387-6396.

It is clear that the mono- (2-)-substituted-4,7disubstituted-1,10-phenanthrolines can be reacted with a different organolithium compound R_{b}-Li affording thus phenanthrolines bearing different substituents in positions 2 and 9.

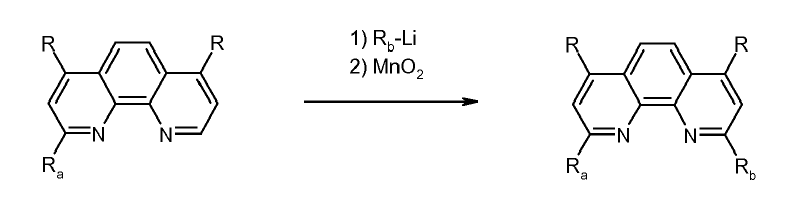

Another possibility for synthesis of the compound of the present invention is the Suzuki coupling of the suitable halo-substituted phenanthroline with appropriate boronic acid. Syntheses of this type and the use of the resulting phenanthrolines in organic light emitting devices are described in, e.g., WO 2004/005288/ A2, WO 2004/006352/ A2 and US 2007/122657/A1.

Of course, the compounds of formula (I) or (II) can be further modified by methods commonly known in the art of organic synthesis.

Preferred is a compound of formula (I) or (II), wherein
in formula (I) R₁ and R₂ are independently substituted or unsubstituted C₆-C₂₀ aryl, and
in formula (II) R₃ is C₃-C₁₂ heteroaryl, R₄ is H, C₆- C₁₂ alkyl, C₆-C₂₅ aryl, or C₃-C₁₂ heteroaryl, and R₅ is C₆-C₁₂ aryl or C₃-C₁₂ heteroaryl.

Particularly preferred is a compound of formula (I) or (II), wherein
aryl is selected from phenyl, biphenylyl, naphthyl, fluorenyl, anthrenyl, anthracenyl, phenanthrenyl, perylenyl, and pyrenyl;
heteroaryl is selected from pyrryl, thienyl, triazolyl, pyrazolyl, oxazolyl, imidazolyl, pyridyl, pyrimidyl, pyridazyl, triazinyl, chinolyl, isochinolyl, carbazolyl, quinazolinyl, quinoxylinyl, phenazinyl, phthalazinyl, acridinyl, pteridyl, benzimidazolyl, benzofuranyl, benzoxazolyl, xanthenyl, phenoxazinyl, and phenothiazinyl; and
the optional substituents on R₁ to R₅ independently are selected from C₁- C₁₈ alkyl, C₄-C₁₂ cycloalkyl, halogen, cyano, C₁-C₁₈ alkoxy, -COO-C₁-C₁₈ alkyl, -CONH₂, -CO-NH-C₁-C₁₈ alkyl, -CO-N(C₁-C₁₈ alkyl)₂, -CO-NH-C₆-C₂₅ aryl, -CO-N(C₆-C₂₆ aryl)₂, -NH₂, -NH-C₁-C₁₈ alkyl, - N(C₁-C₁₈ alkyl)₂, -NH- C₆-C₂₅ aryl, -N(C₆-C₂₆ aryl)₂.

Examples of phenanthrolines of formula (Ia) are listed below:

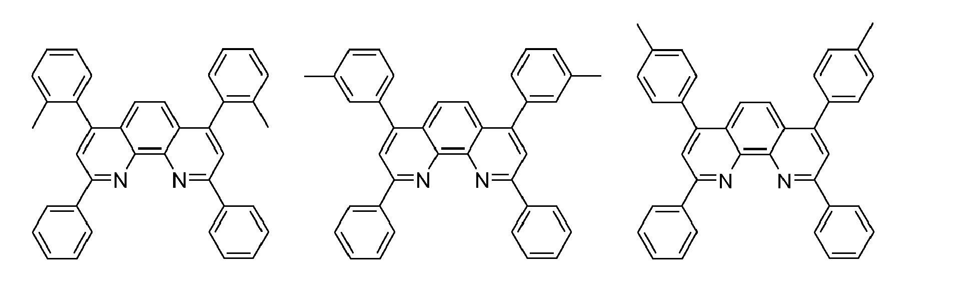

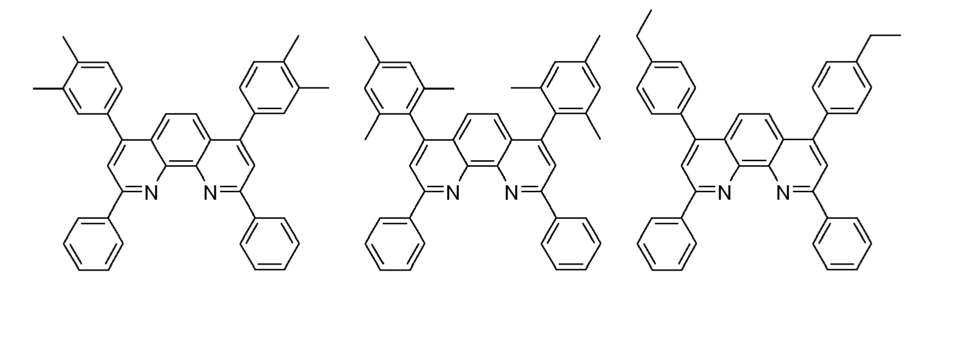

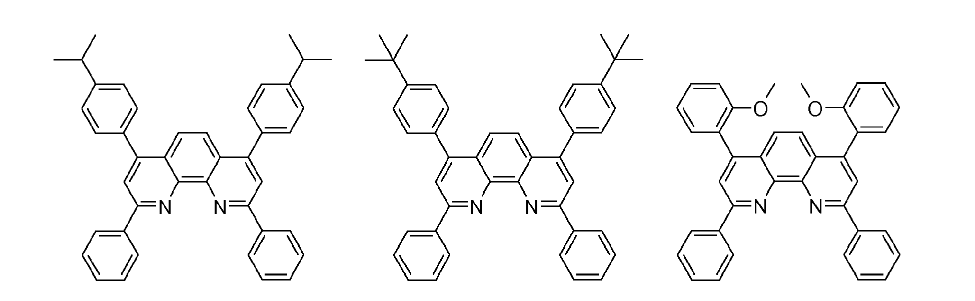

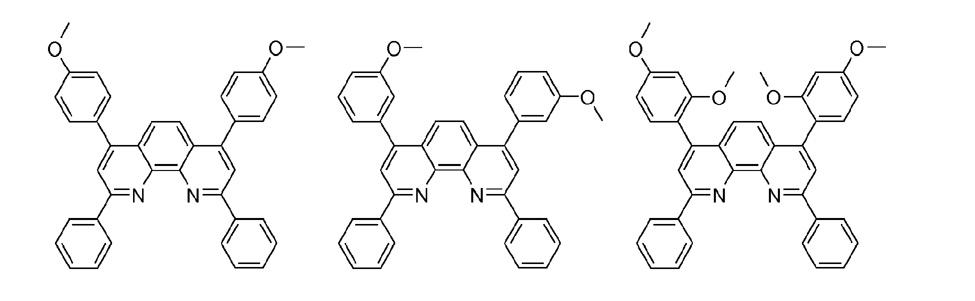

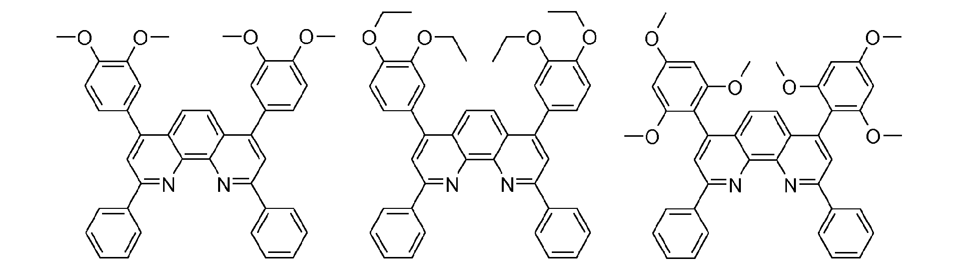

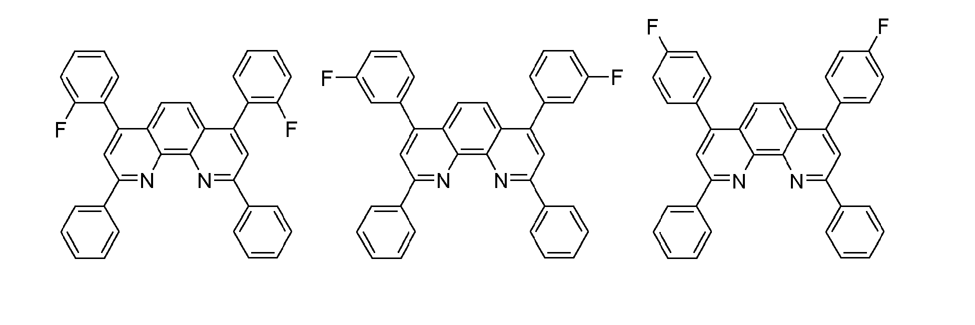

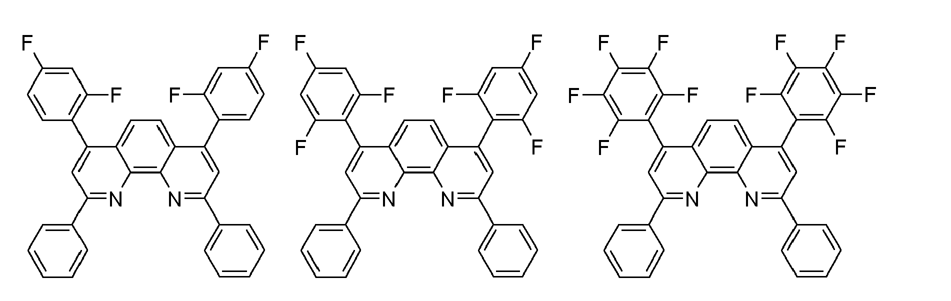

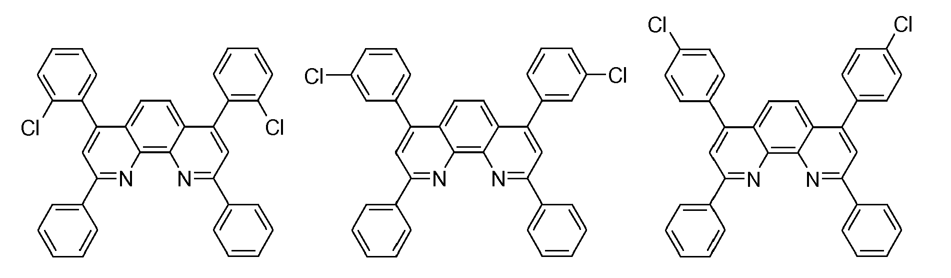

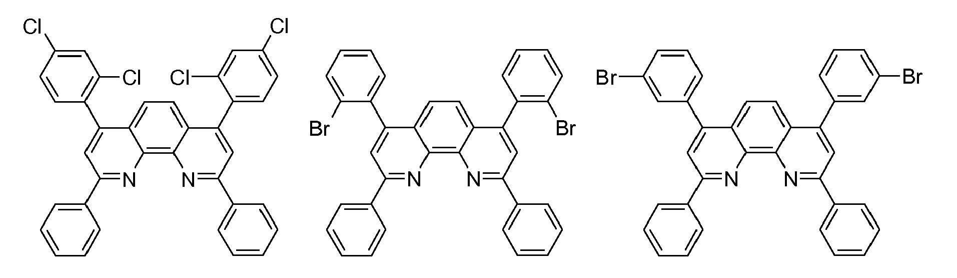

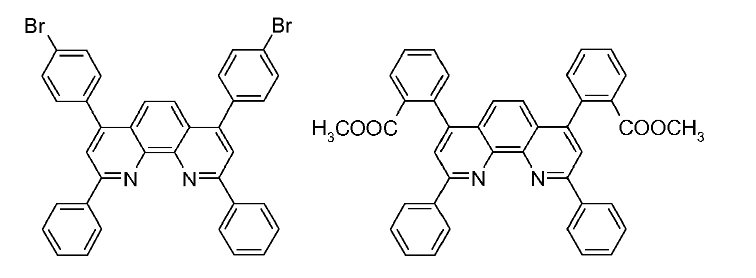

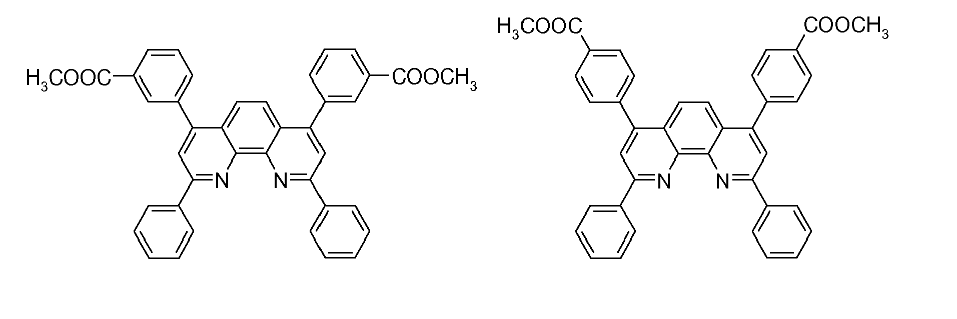

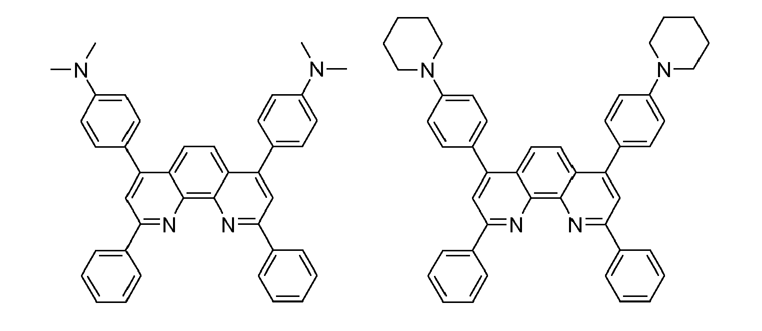

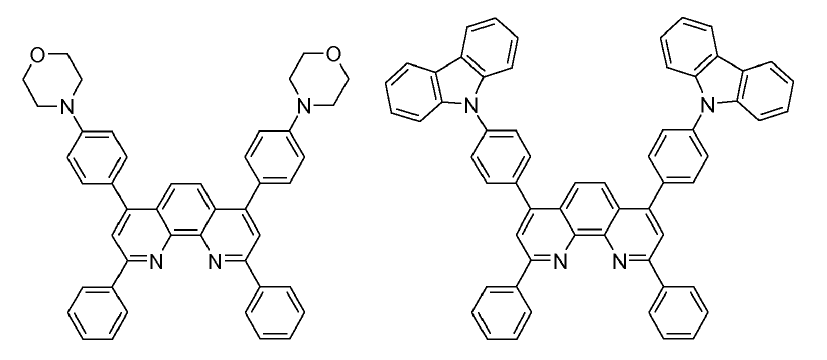

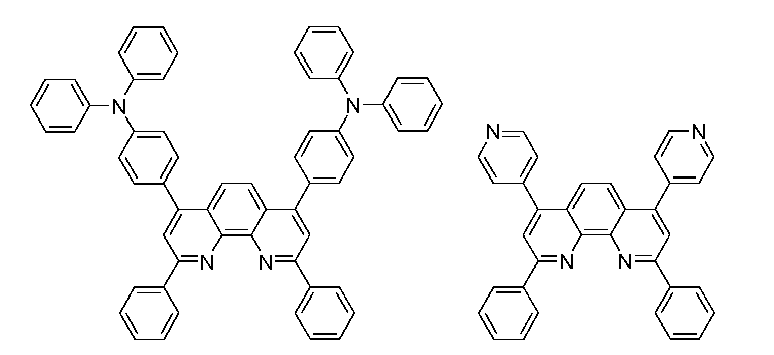

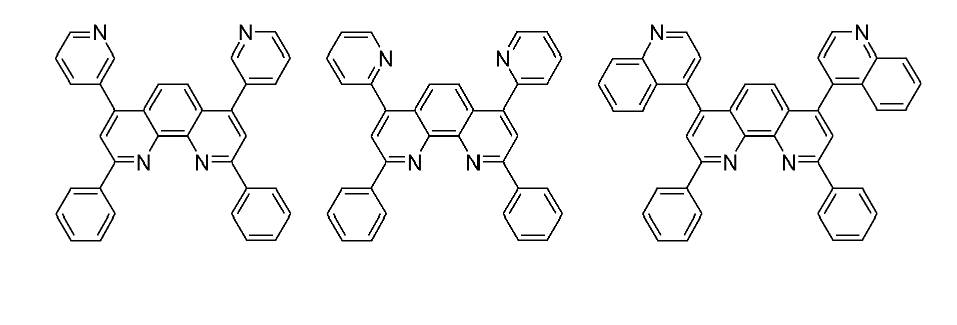

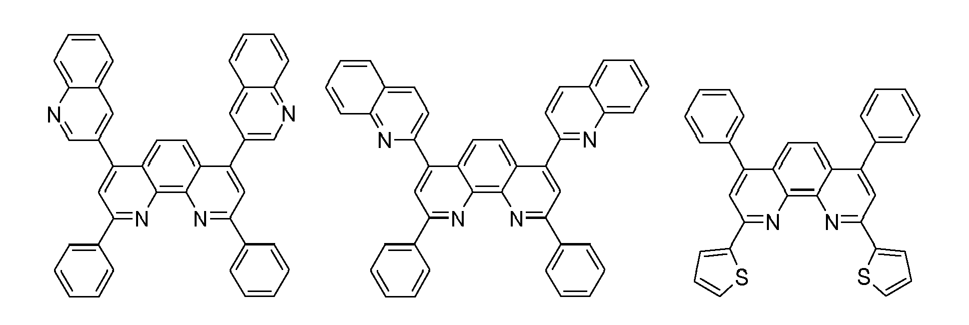

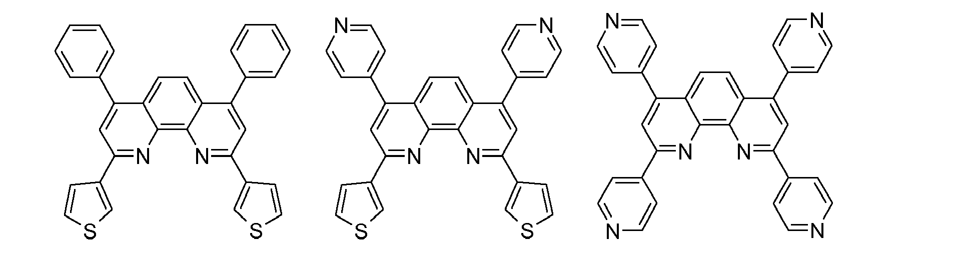

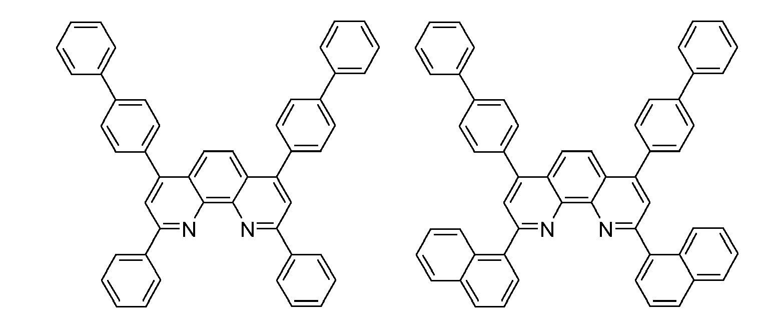

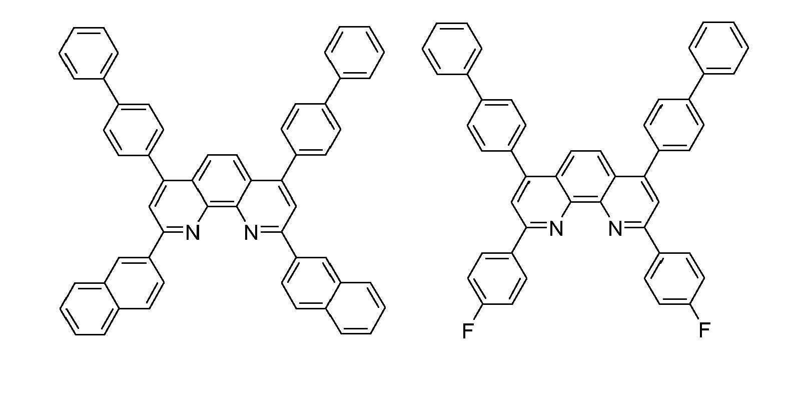

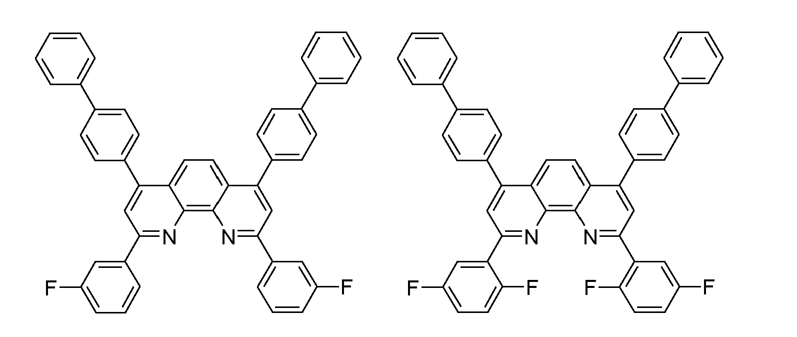

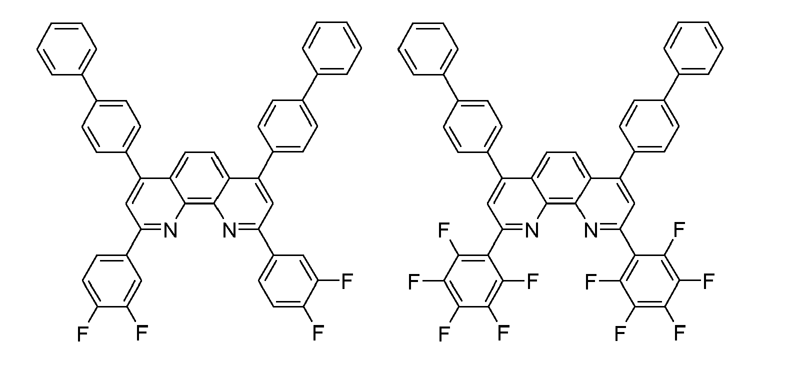

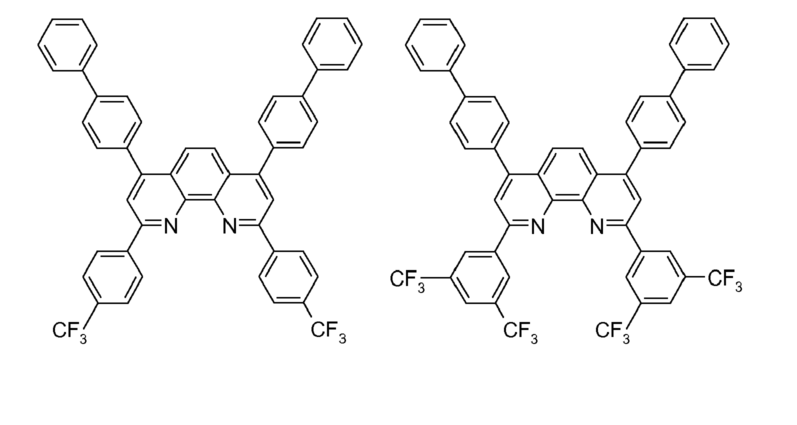

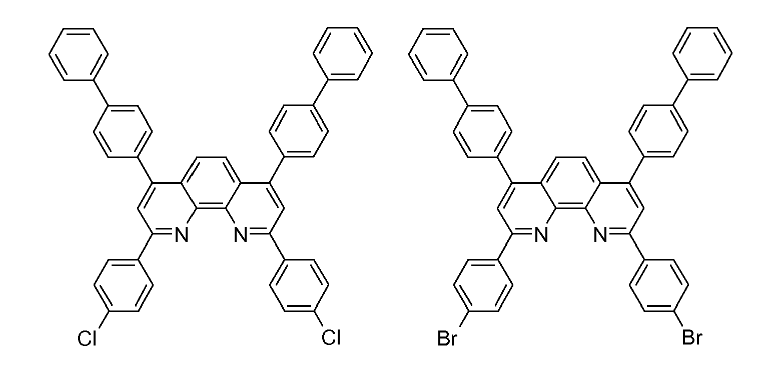

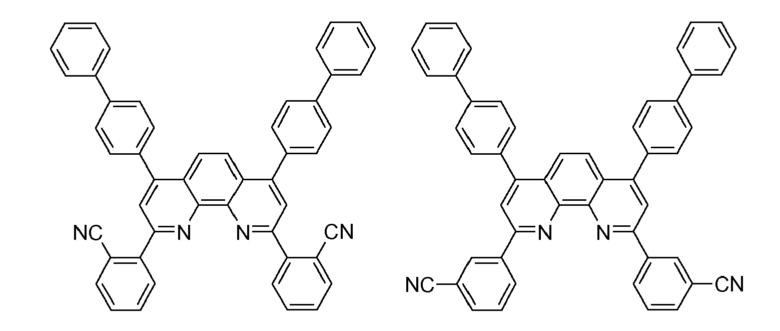

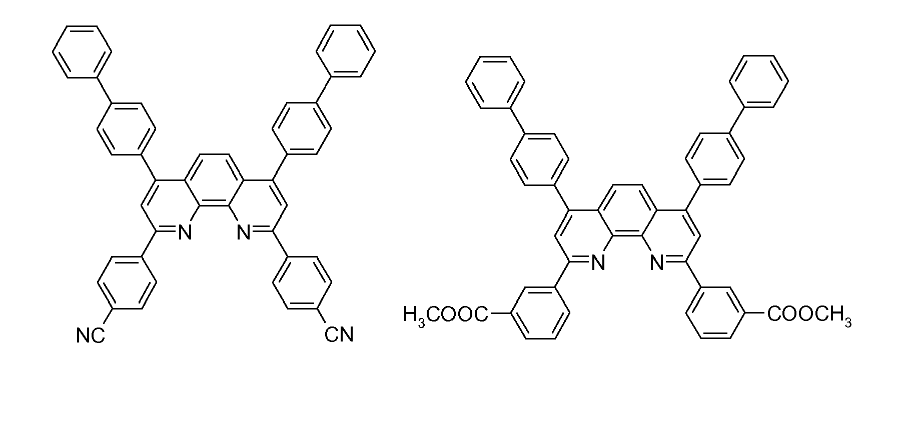

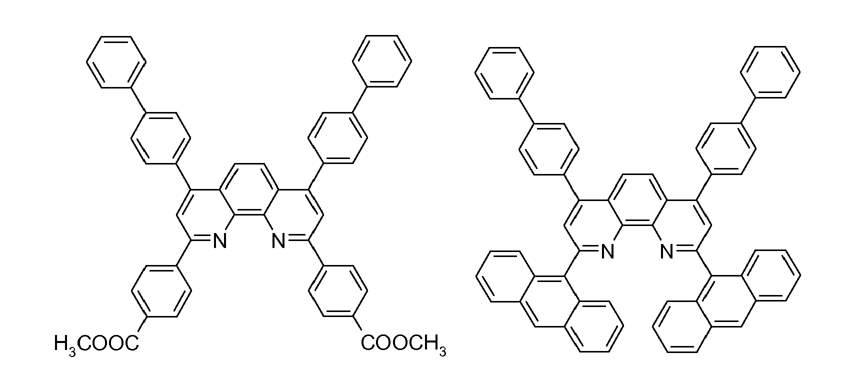

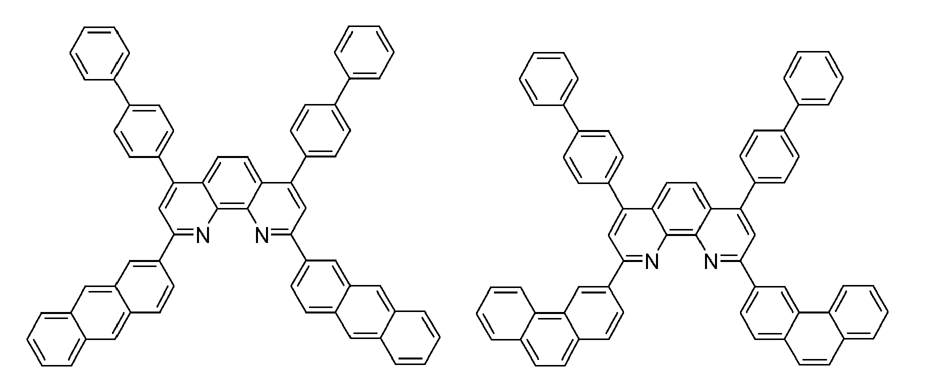

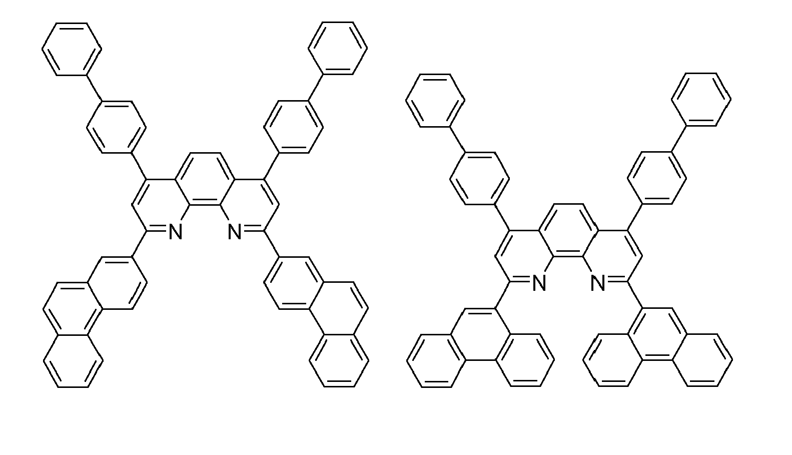

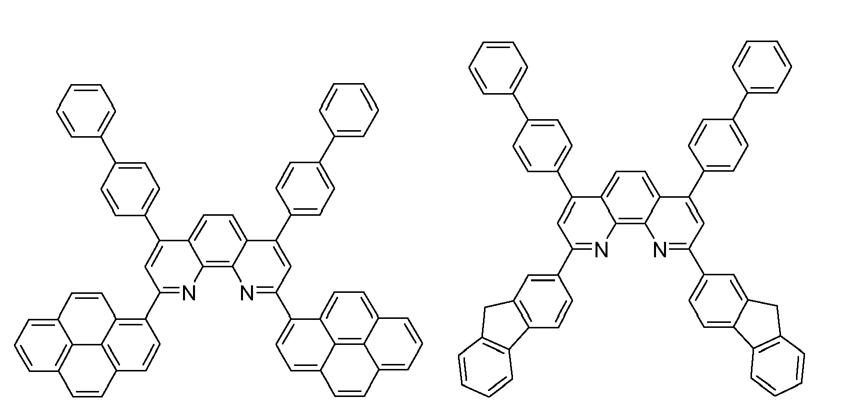

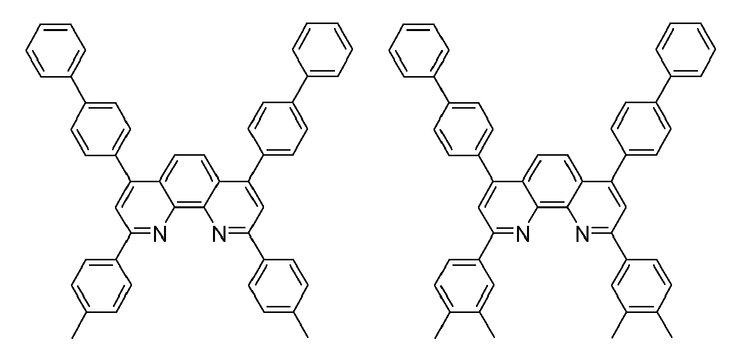

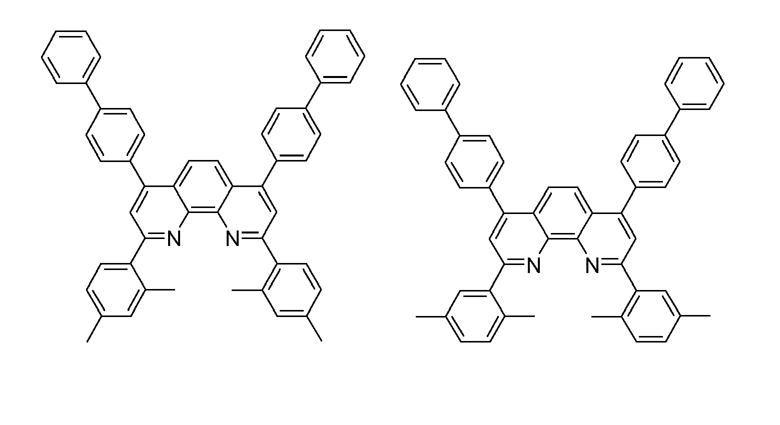

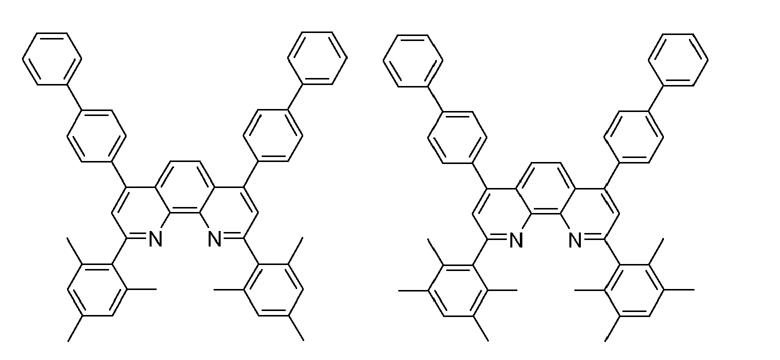

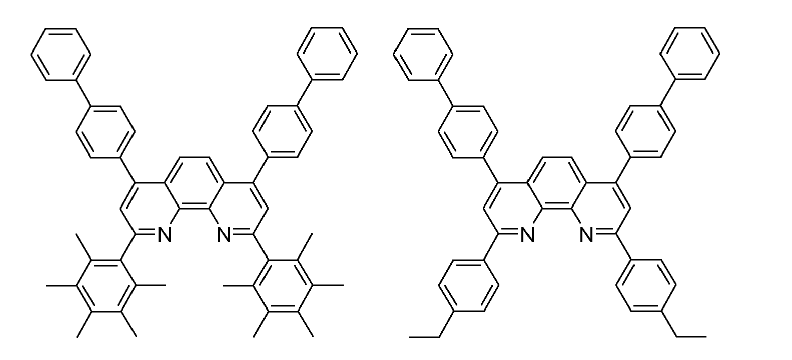

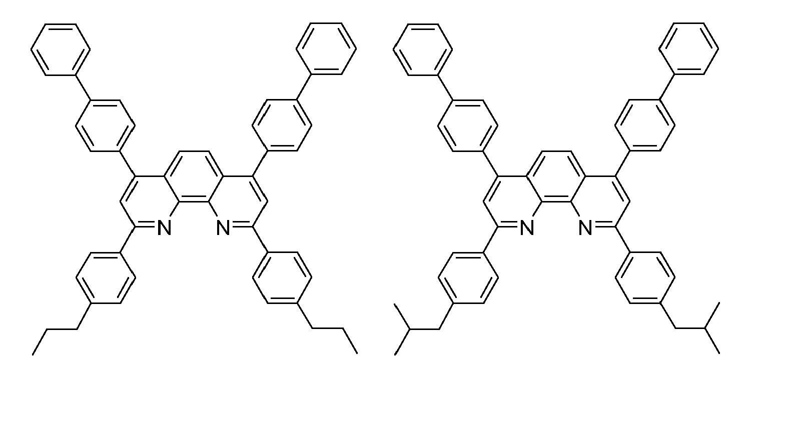

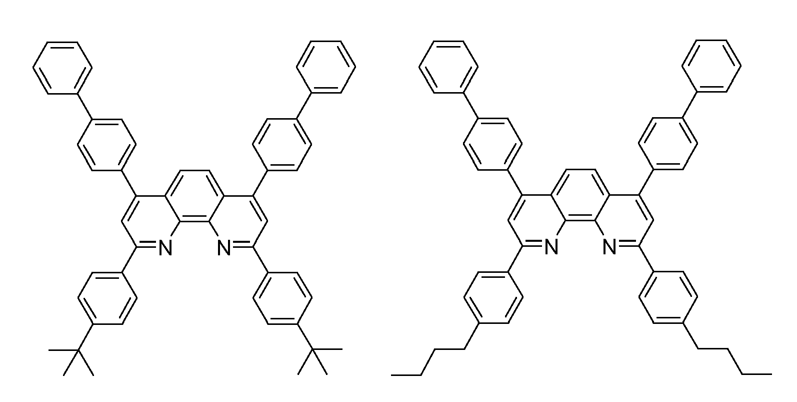

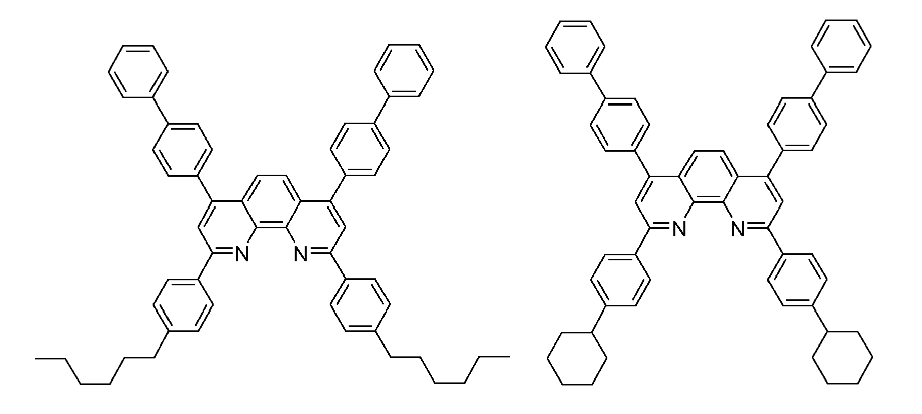

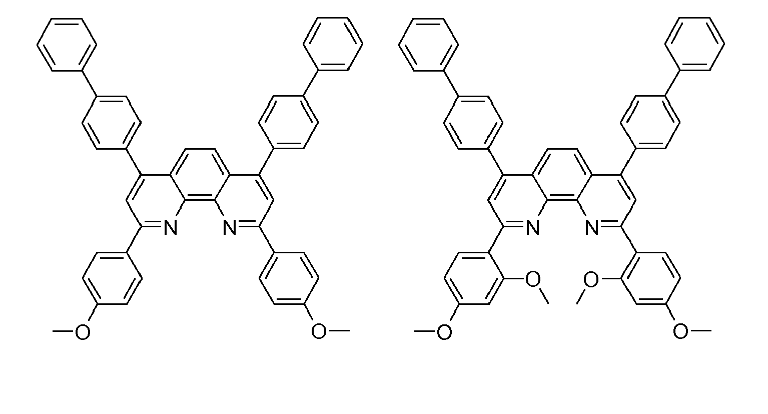

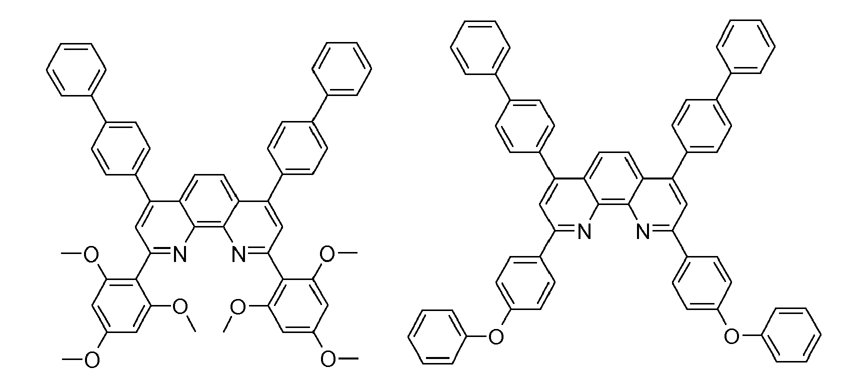

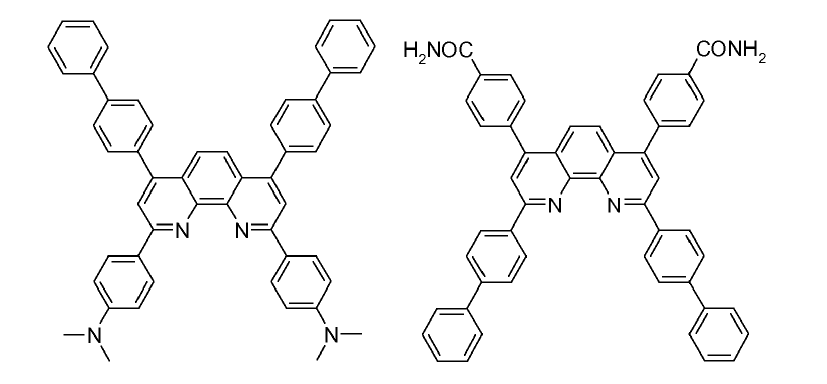

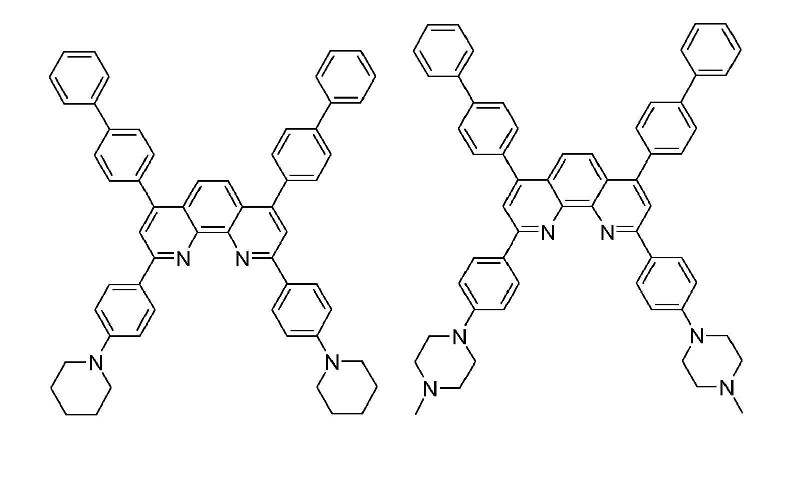

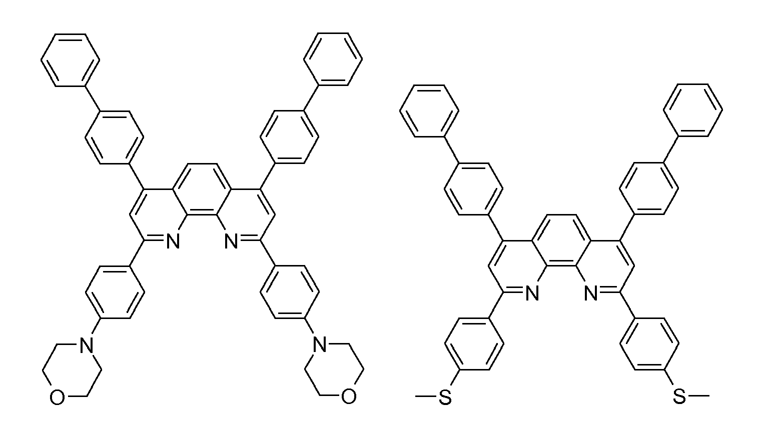

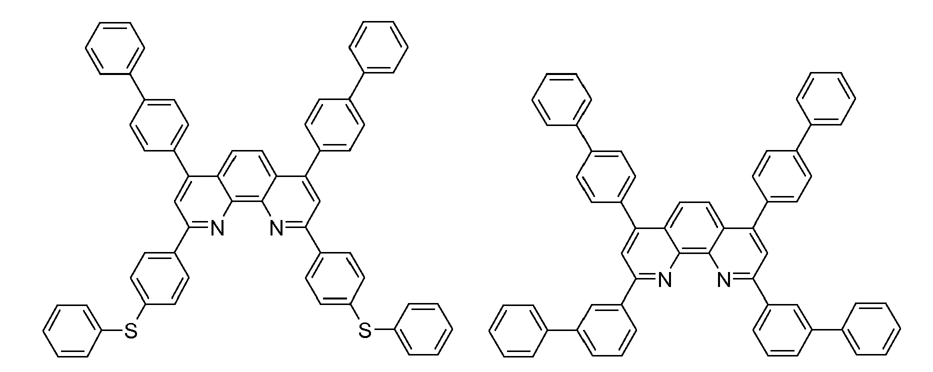

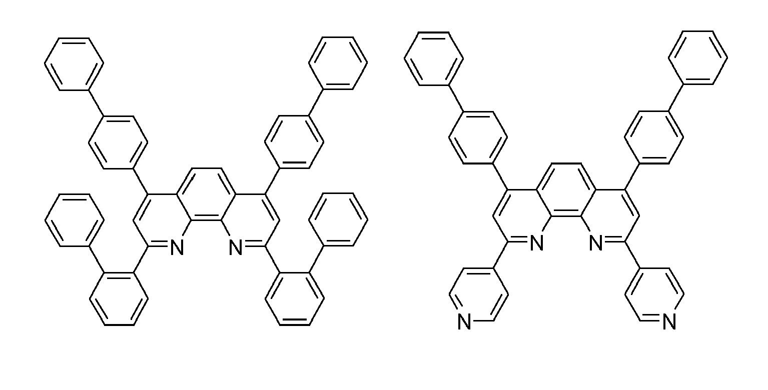

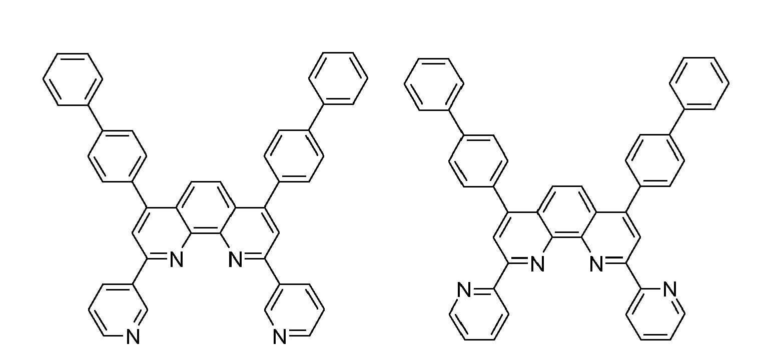

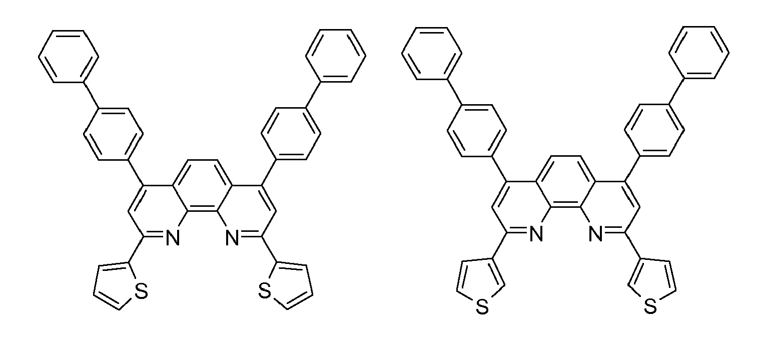

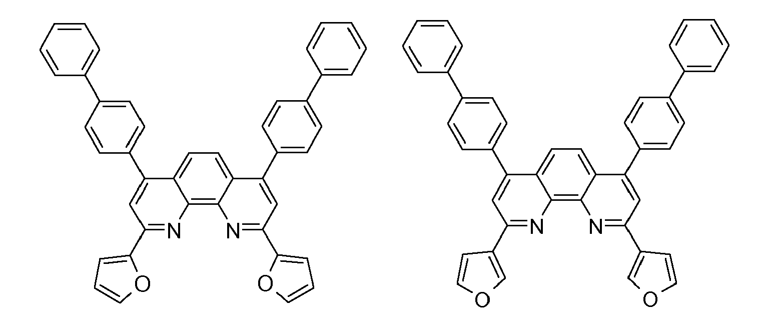

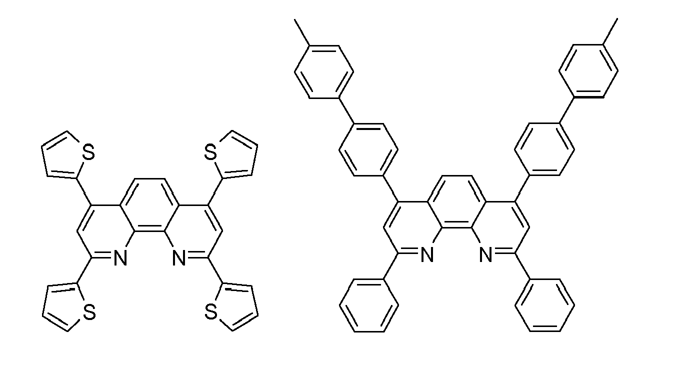

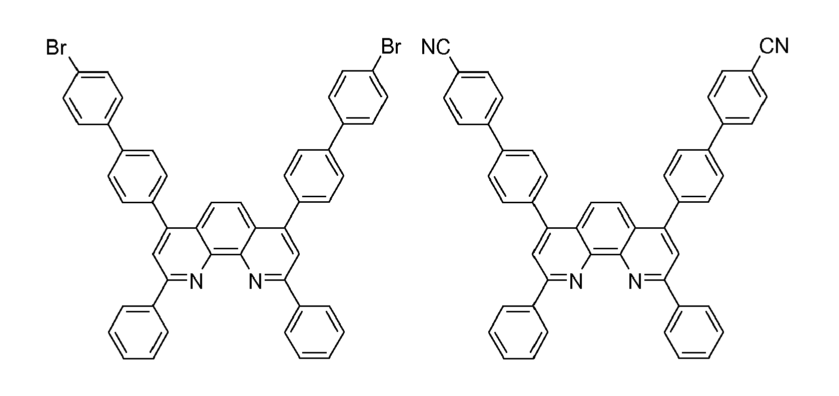

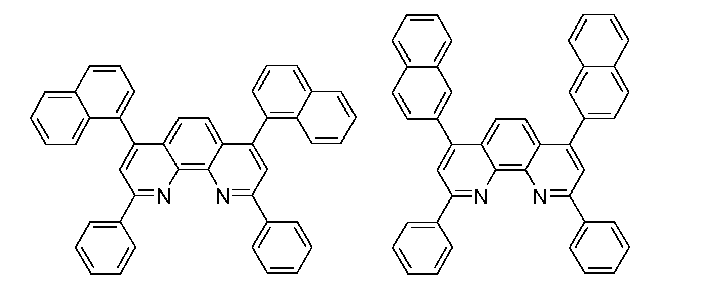

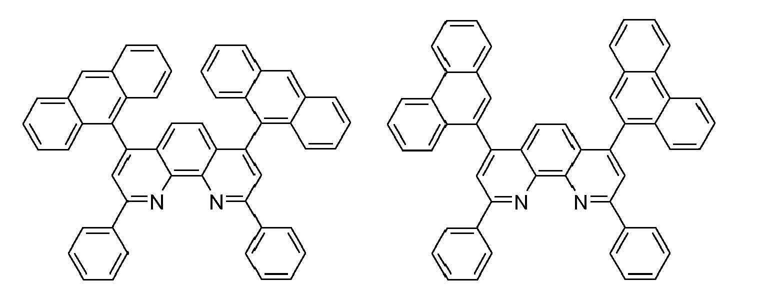

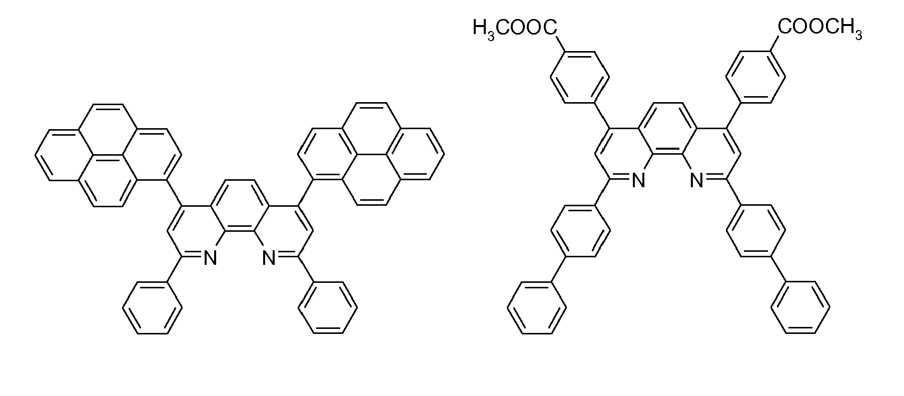

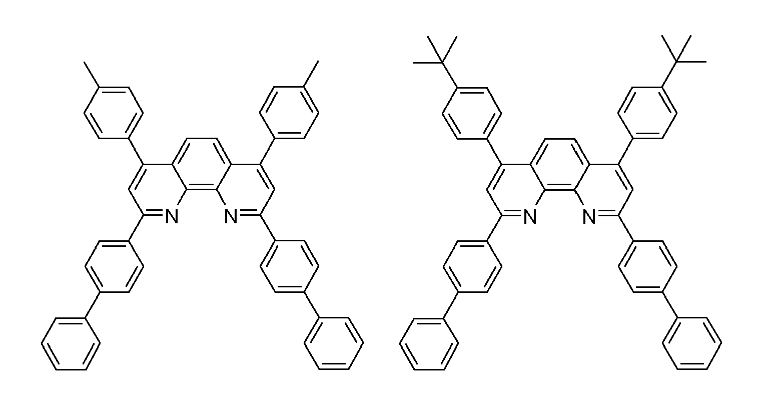

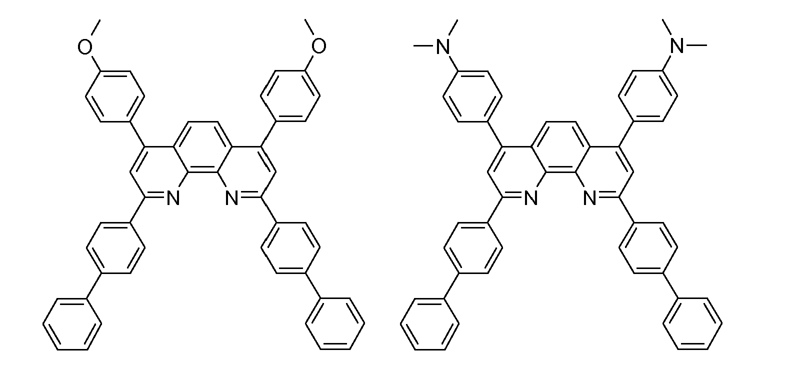

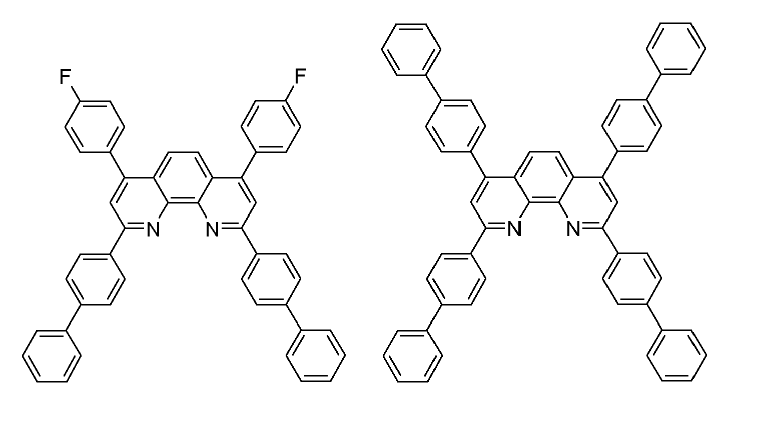

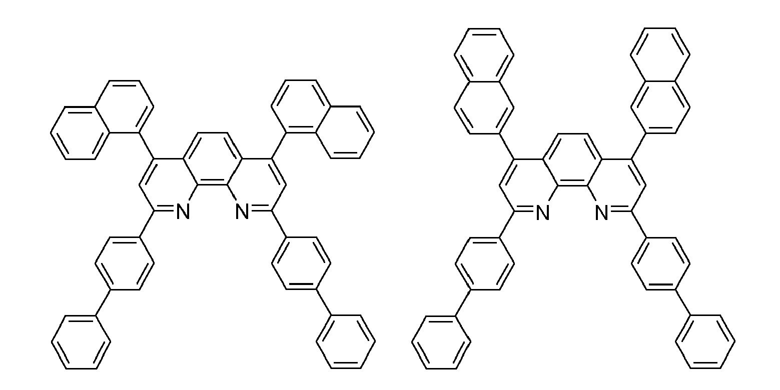

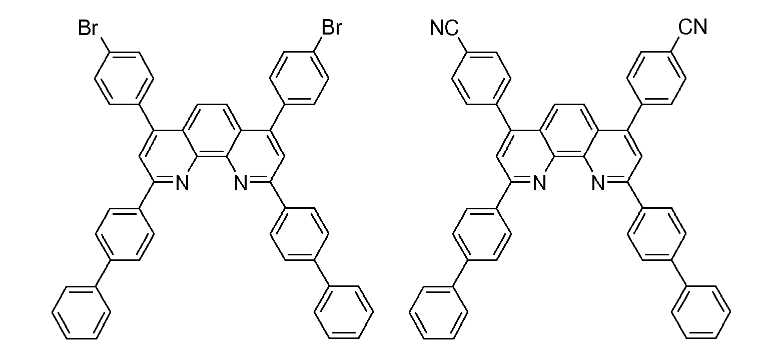

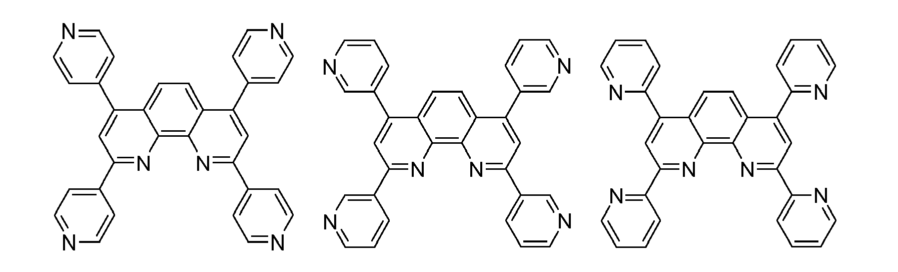

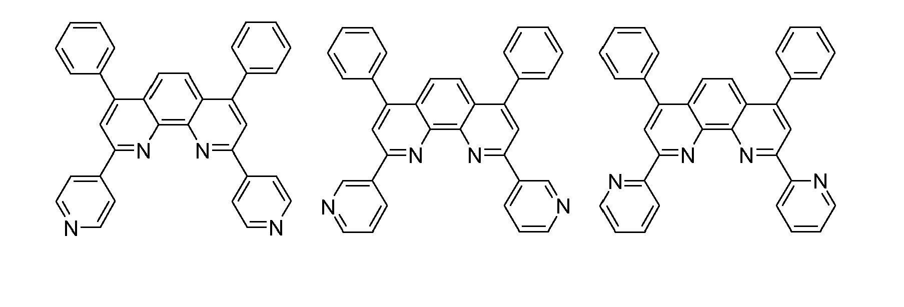

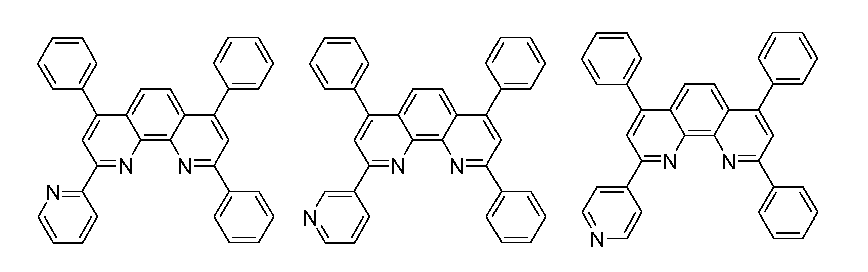

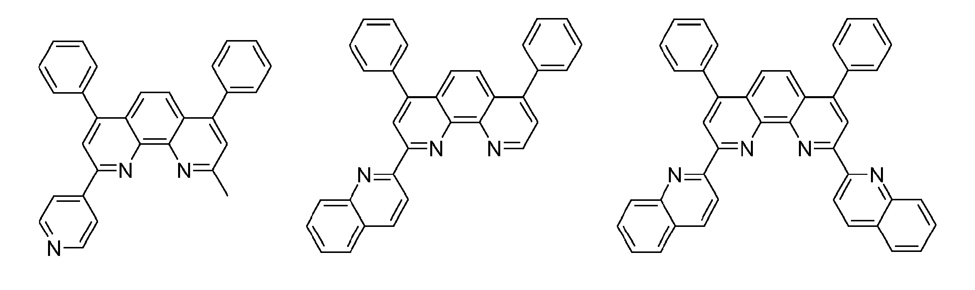

The compounds of formula (I) or (II) can be used in organic light emitting diodes (OLEDs), especially as electron transport layers, as hole blocking layers and/or as hosts for phosphorescent emitter compounds.

Accordingly, another embodiment of the present invention is an electroluminescent (EL) device, comprising a compound of formula (I) or (II) as defined herein. For instance, the electroluminescent device comprises a cathode, an anode, and there between a light emitting layer containing a host material and a phosphorescent light-emitting material, wherein the compound of formula (I) or (II) is the host material.

For instance, an electroluminescent device contains an electron transport layer comprising the compound of formula (I) or (II).For example, an electroluminescent device contains a hole blocking layer comprising the compound of formula (I) or (II).

Another embodiment is the use of a compound of formula (I) or (II) as defined herein for solar cells, especially dye lasers, in particular electroluminescent devices.

Suitably, the light-emitting layer of the OLED device comprises a host material and one or more guest materials for emitting light. At least one of the host materials is a compound of formula (I) or (II). The light-emitting guest material(s) is usually present in an amount less than the amount of host materials and is typically present in an amount of up to 25 wt % of the host, more typically from 0.1 to 20 wt % of the host, and commonly from 8 to 15 wt % of the host. For convenience, the phosphorescent complex guest material may be referred to herein as a phosphorescent material. The emissive layer may comprise a single material, that combines transport and emissive properties. Whether the emissive material is a dopant or a major constituent, emissive layer may comprise other materials, such as dopants that tune the emission of the emissive layer. The emissive layer may include a plurality of emissive materials capable of, in combination, emitting a desired spectrum of light.

### Other Host Materials for Phosphorescent Materials

The host material useful in the invention may be used alone or in combination with other host materials. Other host materials should be selected so that the triplet exciton can be transferred efficiently from the host material to the phosphorescent material. Suitable host materials are described in WO00/70655; 01/39234; 01/93642; 02/074015; 02/15645, and US20020117662. Suitable hosts include certain aryl amines, triazoles, indoles and carbazole compounds. Examples of hosts are 4,4'-N,N'-dicarbazole-biphenyl (CBP), 2,2'-dimethyl-4,4'-N,N'-dicarbazole-biphenyl, m-(N,N'-dicarbazole)benzene, and poly(N-vinylcarbazole), including their derivatives.
Desirable host materials are capable of forming a continuous film. The light-emitting layer may contain more than one host material in order to improve the device's film morphology, electrical properties, light emission efficiency, and lifetime. The light emitting layer may contain a first host material that has good hole-transporting properties, and a second host material that has good electron-transporting properties.

### Phosphorescent Materials

Phosphorescent materials may be used alone or, in certain cases, in combination with each other, either in the same or different layers. Examples of phosphorescent and related materials are described in WO00/57676, WO00/70655, WO01/41512, WO02/15645, US2003/0017361, WO01/93642, WO01/39234, US6,458,475, WO02/071813, US6,573,651, US2002/0197511, WO02/074015, US6,451,455, US2003/0072964, US2003/0068528, US6,413,656, 6,515,298, 6,451,415, 6,097,147, US2003/0124381, US2003/0059646, US2003/0054198, EP1239526, EP1238981, EP1244155, US2002/0100906, US2003/0068526, US2003/0068535, JP2003073387, JP2003073388, US2003/0141809, US2003/0040627, JP2003059667, JP2003073665 and US2002/0121638.

The emission wavelengths of cyclometallated Ir(III) complexes of the type IrL₃ and IrL₂L', such as the green-emitting fac-tris(2-phenylpyridinato-N,C^{2'})iridium(III) and bis(2-phenylpyridinato-N,C^{2'})Iridium(III) (acetylacetonate) may be shifted by substitution of electron donating or withdrawing groups at appropriate positions on the cyclometallating ligand L, or by choice of different heterocycles for the cyclometallating ligand L. The emission wavelengths may also be shifted by choice of the ancillary ligand L'. Examples of red emitters are the bis(2-(2'-benzothienyl)pyridinato-N,C^{3'})iridium(EI)(acetylacetonate) and tris(1-phenylisoquinolinato-N,C)iridium(III). A blue-emitting example is bis(2-(4,6-diflourophenyl)-pyridinato-N,C^{2'})Iridium(III)(picolinate).

Red electrophosphorescence has been reported, using bis(2-(2'-benzo[4,5-a]thienyl)pyridinato-N, C³)iridium(acetylacetonate)[Btp₂Ir(acac)] as the phosphorescent material (Adachi, C., Lamansky, S., Baldo, M. A., Kwong, R. C., Thompson, M. E., and Forrest, S. R., App. Phys. Lett., 78, 1622 1624 (2001).

Other important phosphorescent materials include cyclometallated Pt(II) complexes such as cis-bis(2-phenylpyridinato-N,C^{2'})platinum(II), cis-bis(2-(2'-thienyl)pyridinato-N,C^{3'}) platinum(II), cis-bis(2-(2'-thienyl)qu inolinato-N,C^{5'}) platinum(II), or (2-(4,6-diflourophenyl)pyridinato-NC2') platinum(II)acetylacetonate. Pt(II)porphyrin complexes such as 2,3,7,8,12,13,17,18-octaethyl-21H, 23H-porphine platinum(H) are also useful phosphorescent materials.

Still other examples of useful phosphorescent materials include coordination complexes of the trivalent lanthanides such as Th³⁺ and Eu³⁺ (J. Kido et al, Appl. Phys. Lett., 65, 2124 (1994)).

Other important phosphorescent materials are described in WO06/000544 and European patent application no. 07102949.0.

Examples of phosphorescent materials are compounds A-1 to A-44, B-1 to B-234, C-1 to C-44 and D-1 to D-279, which are described in European patent application no. 07102949.0.

### Blocking Layers

In addition to suitable hosts, an OLED device employing a phosphorescent material often requires at least one electron or hole blocking layers to help confine the electron-hole recombination centers to the light-emitting layer comprising the host and phosphorescent material, or to reduce the number of charge carriers (electrons or holes). In one embodiment, such a blocking layer would be placed between the electron-transporting layer and the light-emitting layer. In this case, the ionization potential of the blocking layer should be such that there is an energy barrier for hole migration from the host into the electron-transporting layer, while the electron affinity should be such that electrons pass more readily from the electron-transporting layer into the light-emitting layer comprising host and phosphorescent material. It is further desired, but not absolutely required, that the triplet energy of the blocking material be greater than that of the phosphorescent material. Suitable hole-blocking materials are described in WO00/70655 and WO01/93642. Two examples of useful materials are bathocuproine (BCP) and bis(2-methyl-8-quinolinolato)(4-phenylphenolato)aluminum(III) (BAIQ). Metal complexes other than Balq are also known to block holes and electrons as described in US20030068528. US20030175553 describes the use of fac-tris(1-phenylpyrazolato-N,C 2)iridium(III) (Irppz) in an electron blocking layer.

Embodiments of the invention can provide advantageous features such as higher operating efficiency, luminance and lifetime with improved color point, lower driving voltage, and improved operational stability. Embodiments of the organometallic compounds useful in the invention can provide a wide range of hues including those useful in the emission of white light (directly or through filters to provide multicolor displays).

### General Device Architecture

The compounds of the present invention can be employed in many OLED device configurations using small molecule materials, oligomeric materials, polymeric materials, or combinations thereof. These include very simple structures comprising a single anode and cathode to more complex devices, such as passive matrix displays comprised of orthogonal arrays of anodes and cathodes to form pixels, and active-matrix displays where each pixel is controlled independently, for example, with thin film transistors (TFTs).

There are numerous configurations of the organic layers. The essential requirements of an OLED are an anode, a cathode, and an organic light-emitting layer located between the anode and cathode. Additional layers may be employed as more fully described hereafter.

A typical structure, especially useful for a small molecule device, is comprised of a substrate, an anode, a hole-injecting layer, a hole-transporting layer, a light-emitting layer, a hole- and/or electron blocking layer, an electron-transporting layer, and a cathode. These layers are described in detail below. Note that the substrate may alternatively be located adjacent to the cathode, or the substrate may actually constitute the anode or cathode. The organic layers between the anode and cathode are conveniently referred to as the organic EL element. Also, the total combined thickness of the organic layers is desirably less than 500 nm.

### Substrate

The substrate can either be light transmissive or opaque, depending on the intended direction of light emission. The light transmissive property is desirable for viewing the EL emission through the substrate. Transparent glass or plastic is commonly employed in such cases. The substrate can be a complex structure comprising multiple layers of materials. This is typically the case for active matrix substrates wherein TFTs are provided below the OLED layers. It is still necessary that the substrate, at least in the emissive pixilated areas, be comprised of largely transparent materials such as glass or polymers. For applications where the EL emission is viewed through the top electrode, the transmissive characteristic of the bottom support is immaterial, and therefore can be light transmissive, light absorbing or light reflective. Substrates for use in this case include, but are not limited to, glass, plastic, semiconductor materials, silicon, ceramics, and circuit board materials. Again, the substrate can be a complex structure comprising multiple layers of materials such as found in active matrix TFT designs. It is necessary to provide in these device configurations a light-transparent top electrode.

### Anode

When the desired electroluminescent light emission (EL) is viewed through the anode, the anode should be transparent or substantially transparent to the emission of interest. Common transparent anode materials used in this invention are indium-tin oxide (ITO), indium-zinc oxide (IZO) and tin oxide, but other metal oxides can work including, but not limited to, aluminum- or indium-doped zinc oxide, magnesium-indium oxide, and nickel-tungsten oxide. In addition to these oxides, metal nitrides, such as gallium nitride, and metal selenides, such as zinc selenide, and metal sulfides, such as zinc sulfide, can be used as the anode. For applications where EL emission is viewed only through the cathode, the transmissive characteristics of the anode are immaterial and any conductive material can be used, transparent, opaque or reflective. Example conductors for this application include, but are not limited to, gold, iridium, molybdenum, palladium, and platinum. Desired anode materials are commonly deposited by any suitable means such as evaporation, sputtering, chemical vapor deposition, or electrochemical means. Anodes can be patterned using well-known photolithographic processes. Optionally, anodes may be polished prior to application of other layers to reduce surface roughness so as to minimize shorts or enhance reflectivity.

### Cathode

When light emission is viewed solely through the anode, the cathode used in this invention can be comprised of nearly any conductive material. Desirable materials have good film-forming properties to ensure good contact with the underlying organic layer, promote electron injection at low voltage, and have good stability. Useful cathode materials often contain a low work function metal (<4.0 eV) or metal alloy. One useful cathode material is comprised of a Mg:Ag alloy wherein the percentage of silver is in the range of 1 to 20%, as described in US-A-4,885,221. Another suitable class of cathode materials includes bilayers comprising the cathode and a thin electron-injection layer (EIL) in contact with an organic layer (e.g., an electron transporting layer (ETL)) which is capped with a thicker layer of a conductive metal. Here, the EIL preferably includes a low work function metal or metal salt, and if so, the thicker capping layer does not need to have a low work function. One such cathode is comprised of a thin layer of LiF followed by a thicker layer of AI as described in US-A-5,677,572. An ETL material doped with an alkali metal, for example, Li-doped Alq, is another example of a useful EIL. Other useful cathode material sets include, but are not limited to, those disclosed in US-A-5,059,861, 5,059,862 and 6,140,763.

When light emission is viewed through the cathode, the cathode must be transparent or nearly transparent. For such applications, metals must be thin or one must use transparent conductive oxides, or a combination of these materials. Optically transparent cathodes have been described in more detail in US-A-4,885,211, 5,247,190, JP 3,234,963, U.S. Pat. Nos. 5,703,436, 5,608,287, 5,837,391, 5,677,572, 5,776,622, 5,776,623, 5,714,838, 5,969,474, 5,739,545, 5,981,306, 6,137,223, 6,140,763, 6,172,459, EP1076368, US-A-6,278,236 and 6,284,393. Cathode materials are typically deposited by any suitable method such as evaporation, sputtering, or chemical vapor deposition. When needed, patterning can be achieved through many well known methods including, but not limited to, through-mask deposition, integral shadow masking as described in US-A-5,276,380 and EP0732868, laser ablation, and selective chemical vapor deposition.

### Hole-Injecting Layer (HIL)

A hole-injecting layer may be provided between anode and hole-transporting layer. The hole-injecting material can serve to improve the film formation property of subsequent organic layers and to facilitate injection of holes into the hole-transporting layer. Suitable materials for use in the hole-injecting layer include, but are not limited to, porphyrinic compounds as described in US-A-4,720,432, plasma-deposited fluorocarbon polymers as described in US-A-6,208,075, and some aromatic amines, for example, m-MTDATA (4,4',4"-tris[(3-methylphenyl)phenylamino]triphenylamine). Alternative hole-injecting materials reportedly useful in organic EL devices are described in EP0891121 and EP1029909.

### Hole-Transporting Layer (HTL)

The hole-transporting layer of the organic EL device contains at least one hole-transporting compound such as an aromatic tertiary amine, where the latter is understood to be a compound containing at least one trivalent nitrogen atom that is bonded only to carbon atoms, at least one of which is a member of an aromatic ring. In one form the aromatic tertiary amine can be an arylamine, such as a monoarylamine, diarylamine, triarylamine, or a polymeric arylamine. Exemplary monomeric triarylamines are illustrated in US-A-3,180,730. Other suitable triarylamines substituted with one or more vinyl radicals and/or comprising at least one active hydrogen containing group are disclosed in US-A-3,567,450 and 3,658,520. A more preferred class of aromatic tertiary amines are those which include at least two aromatic tertiary amine moieties as described in US-A-4,720,432 and 5,061,569. Such compounds include those represented by structural formula 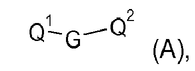 wherein Q¹ and Q² are independently selected aromatic tertiary amine moieties and G is a linking group such as an arylene, cycloalkylene, or alkylene group of a carbon to carbon bond. In one embodiment, at least one of Q¹ or Q² contains a polycyclic fused ring structure, e.g., a naphthalene. When G is an aryl group, it is conveniently a phenylene, biphenylene, or naphthalene moiety.
A useful class of triarylamines satisfying structural formula (A) and containing two triarylamine moieties is represented by structural formula 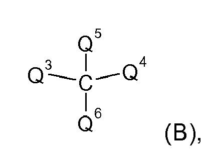 where Q³ and Q⁴ each independently represents a hydrogen atom, an aryl group, or an alkyl group or Q³ and Q⁴ together represent the atoms completing a cycloalkyl group; and Q⁵ and Q⁶ each independently represents an aryl group, which is in turn substituted with a diaryl substituted amino group, as indicated by structural formula 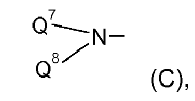 wherein Q⁷ and Q⁸ are independently selected aryl groups. In one embodiment, at least one of Q⁷ or Q⁸ contains a polycyclic fused ring structure, e.g., a naphthalene.

Another class of aromatic tertiary amines are the tetraaryldiamines. Desirable tetraaryldiamines include two diarylamino groups, such as indicated by formula (C), linked through an arylene group. Useful tetraaryldiamines include those represented by formula 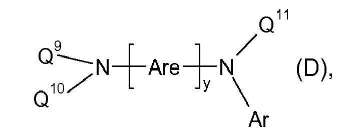 wherein each Are is an independently selected arylene group, such as a phenylene or anthracene moiety, n is an integer of from 1 to 4, and Ar, Q⁹, Q¹⁰, and Q¹¹ are independently selected aryl groups. In a typical embodiment, at least one of Ar, Q⁹, Q¹⁰, and Q¹¹ is a polycyclic fused ring structure, e.g., a naphthalene. The various alkyl, alkylene, aryl, and arylene moieties of the foregoing structural formulae (A), (B), (C), (D), can each in turn be substituted. Typical substituents include alkyl groups, alkoxy groups, aryl groups, aryloxy groups, and halogen such as fluoride, chloride, and bromide. The various alkyl and alkylene moieties typically contain from about 1 to 6 carbon atoms. The cycloalkyl moieties can contain from 3 to about 10 carbon atoms, but typically contain five, six, or seven ring carbon atoms, e.g. cyclopentyl, cyclohexyl, and cycloheptyl ring structures. The aryl and arylene moieties are usually phenyl and phenylene moieties.

The hole-transporting layer can be formed of a single or a mixture of aromatic tertiary amine compounds. Specifically, one may employ a triarylamine, such as a triarylamine satisfying the formula (B), in combination with a tetraaryldiamine, such as indicated by formula (D). When a triarylamine is employed in combination with a tetraaryldiamine, the latter is positioned as a layer interposed between the triarylamine and the electron injecting and transporting layer. Illustrative of useful aromatic tertiary amines are the following: 1,1-Bis(4-di-p-tolylaminophenyl)cyclohexane, 1,1-bis(4-di-p-tolylaminophenyl)-4-phenylcyclohexane, N,N,N',N'-tetraphenyl-4,4"'-diamino-1,1':4',1":4", 1"'-quaterphenyl bis(4-dimethylamino-2-methylphenyl)phenylmethane, 1,4-bis[2-[4-[N,N-di(p-toly)amino]phenyl]vinyl]benzene (BDTAPVB), N,N,N',N'-tetra-p-tolyl-4,4'-diaminobiphenyl, N,N,N',N'-tetraphenyl-4,4'-diaminobiphenyl, N,N,N',N'-tetra-1-naphthyl-4,4'-diaminobiphenyl, N,N,N',N'-tetra-2-naphthyl-4,4'-diaminobiphenyl, N-phenylcarbazole, 4,4'-bis[N-(1-naphthyl)-N-phenylamino]biphenyl (NPB), 4,4'-bis[N-(1-naphthyl)-N-(2-naphthyl)amino]biphenyl (TNB), 4,4'-bis[N-(1-naphthyl)-N-phenylamino]p-terphenyl, 4,4'-bis[N-(2-naphthyl)-N-phenylamino]biphenyl, 4,4'-bis[N-(3-acenaphthenyl)-N-phenylamino]biphenyl, 1,5-bis[N-(1-naphthyl)-N-phenylamino] naphthalene, 4,4'-bis[N-(9-anthryl)-N-phenylamino]biphenyl, 4,4'-bis[N-(1-anthryl)-N-phenylamino]-p-terphenyl, 4,4'-bis[N-(2-phenanthryl)-N-phenylamino]biphenyl, 4,4'-bis[N-(8-fluoranthenyl)-N-phenylamino]biphenyl, 4,4'-bis[N-(2-pyrenyl)-N-phenylamino]biphenyl, 4,4'-bis[N-(2-naphthacenyl)-N-phenylamino]biphenyl, 4,4'-bis[N-(2-perylenyl)-N-phenylamino] biphenyl, 4,4'-bis[N-(1-coronenyl)-N-phenylamino]biphenyl, 2,6-bis(di-p-tolylamino) naphthalene, 2,6-bis[di-(1-naphthyl)amino]naphthalene, 2,6-bis[N-(1-naphthyl)-N-(2-naphthyl)amino]naphthalene, N,N,N',N'-tetra(2-naphthyl)-4,4"-diamino-p-terphenyl, 4,4'-bis {N-phenyl-N-[4-(1-naphthyl)-phenyl]amino}biphenyl, 2,6-bis[N,N-di(2-naphthyl)amino]fluorine, 4,4',4"-tris[(3-methylphenyl)phenylamino]triphenylamine (MTDATA), and 4,4'-Bis[N-(3-methylphenyl)-N-phenylamino]biphenyl(TPD). A hole transport layer may be used to enhance conductivity. NPD and TPD are examples of intrinsic hole transport layers. An example of a p-doped hole transport layer is m-MTDATA doped with F₄-TCNQ at a molar ratio of 50:1 as disclosed in US6,337,102 or DE10058578.

Another class of useful hole-transporting materials includes polycyclic aromatic compounds as described in EP1009041. Tertiary aromatic amines with more than two amine groups may be used including oligomeric materials. In addition, polymeric hole-transporting materials can be used such as poly(N-vinylcarbazole) (PVK), polythiophenes, polypyrrole, polyaniline, and copolymers such as poly(3,4-ethylenedioxythiophene)/poly(4-styrenesulfonate) also called PEDOT/PSS.

### Fluorescent Light-Emitting Materials and Layers (LEL)

In addition to the phosphorescent materials, other light emitting materials may be used in the OLED device, including fluorescent materials. Although the term "fluorescent" is commonly used to describe any light emitting material, in this case we are referring to a material that emits light from a singlet excited state. Fluorescent materials may be used in the same layer as the phosphorescent material, in adjacent layers, in adjacent pixels, or any combination. Care must be taken not to select materials that will adversely affect the performance of the phosphorescent materials. One skilled in the art will understand that triplet excited state energies of materials in the same layer as the phosphorescent material or in an adjacent layer must be appropriately set so as to prevent unwanted quenching. As more fully described in US-A-4,769,292 and 5,935,721, the light-emitting layer (LEL) of the organic EL element includes a luminescent fluorescent or phosphorescent material where electroluminescence is produced as a result of electron-hole pair recombination in this region. The light-emitting layer can be comprised of a single material, but more commonly consists of a host material doped with a guest emitting material or materials where light emission comes primarily from the emitting materials and can be of any color. The host materials in the light-emitting layer can be an electron-transporting material, as defined below, a hole-transporting material, as defined above, or another material or combination of materials that support hole-electron recombination. Fluorescent emitting materials are typically incorporated at 0.01 to 10% by weight of the host material. The host and emitting materials can be small non-polymeric molecules or polymeric materials such as polyfluorenes and polyvinylarylenes (e.g., poly(p-phenylenevinylene), PPV). In the case of polymers, small molecule emitting materials can be molecularly dispersed into a polymeric host, or the emitting materials can be added by copolymerizing a minor constituent into a host polymer. Host materials may be mixed together in order to improve film formation, electrical properties, light emission efficiency, lifetime, or manufacturability. The host may comprise a material that has good hole-transporting properties and a material that has good electron-transporting properties.

Host and emitting materials known to be of use include, but are not limited to, those disclosed in US-A-4,768,292, 5,141,671, 5,150,006, 5,151,629, 5,405,709, 5,484,922, 5,593,788, 5,645,948, 5,683,823, 5,755,999, 5,928,802, 5,935,720, 5,935,721, and 6,020,078.

Metal complexes of 8-hydroxyquinoline and similar derivatives (Formula E) constitute one class of useful host compounds capable of supporting electroluminescence, and are particularly suitable for light emission of wavelengths longer than 500 nm, e.g., green, yellow, orange, and red. 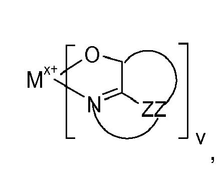 wherein M represents a metal; v is an integer of from 1 to 4; and ZZ independently in each occurrence represents the atoms completing a nucleus having at least two fused aromatic rings. From the foregoing it is apparent that the metal can be monovalent, divalent, trivalent, or tetravalent metal. The metal can, for example, be an alkali metal, such as lithium, sodium, or potassium; an alkaline earth metal, such as magnesium or calcium; an earth metal, such aluminum or gallium, or a transition metal such as zinc or zirconium. Generally any monovalent, divalent, trivalent, or tetravalent metal known to be a useful chelating metal can be employed. ZZ completes a heterocyclic nucleus containing at least two fused aromatic rings, at least one of which is an azole or azine ring. Additional rings, including both aliphatic and aromatic rings, can be fused with the two required rings, if required. To avoid adding molecular bulk without improving on function the number of ring atoms is usually maintained at 18 or less.
Illustrative of useful chelated oxinoid compounds are the following:
CO-1: Aluminum trisoxine [alias, tris(8-quinolinolato)aluminum(III)]
CO-2: Magnesium bisoxine [alias, bis(8-quinolinolato)magnesium(II)]
CO-3: Bis[benzo{f}-8-quinoiinoiato]zinc(ii)
CO-4: Bis(2-methyl-8-quinolinolato)aluminum(III)-µ-oxo-bis(2-methyl-8-quinolinolato)aluminum(III)
CO-5: Indium trisoxine [alias, tris(8-quinolinolato)indium]
CO-6: Aluminum tris(5-methyloxine) [alias, tris(5-methyl-8-quinolinolato) aluminum(III)]
CO-7: Lithium oxine [alias, (8-quinolinolato)lithium(l)]
CO-8: Gallium oxine [alias, tris(8-quinolinolato)gallium(III)]
CO-9: Zirconium oxine [alias, tetra(8-quinolinolato)zirconium(IV)]

Useful fluorescent emitting materials include, but are not limited to, derivatives of anthracene, tetracene, xanthene, perylene, rubrene, coumarin, rhodamine, and quinacridone, dicyanomethylenepyran compounds, thiopyran compounds, polymethine compounds, pyrilium and thiapyrilium compounds, fluorene derivatives, periflanthene derivatives, indenoperylene derivatives, bis(azinyl)amine boron compounds, bis(azinyl)methane compounds, and carbostyryl compounds. Illustrative examples of useful materials include, but are not limited to, compounds L1 to L52 described in US7,090,930B2.

### Electron-Transporting Layer (ETL)

Preferred thin film-forming materials for use in forming the electron-transporting layer of the organic EL devices of this invention are metal chelated oxinoid compounds, including chelates of oxine itself (also commonly referred to as 8-quinolinol or 8-hydroxyquinoline). Such compounds help to inject and transport electrons and exhibit both high levels of performance and are readily fabricated in the form of thin films. Exemplary of contemplated oxinoid compounds are those satisfying structural formula (E), previously described. Other electron-transporting materials include various butadiene derivatives as disclosed in US4,356,429 and various heterocyclic optical brighteners as described in US4,539,507. Benzazoles satisfying structural formula (G) are also useful electron transporting materials. Triazines are also known to be useful as electron transporting materials. Doping may be used to enhance conductivity. Alq₃ is an example of an intrinsic electron transport layer. An example of an n-doped electron transport layer is BPhen doped with Li at a molar ratio of 1:1, as disclosed in US 6,337,102.

### Deposition of Organic Layers

The organic materials mentioned above are suitably deposited by any means suitable for the form of the organic materials. In the case of small molecules, they are conveniently deposited through thermal evaporation, but can be deposited by other means such as from a solvent with an optional binder to improve film formation. If the material is soluble or in oligomeric/polymeric form, solution processing is usually preferred e.g. spin-coating, ink-jet printing. Dendrimer substituents may be used to enhance the ability of small molecules to undergo solution processing. Patterned deposition can be achieved using shadow masks, integral shadow masks (US5,294,870), spatially-defined thermal dye transfer from a donor sheet (US5,688,551, 5,851,709 and 6,066,357) and inkjet method (US6,066,357).

### Encapsulation

Most OLED devices are sensitive to moisture or oxygen, or both, so they are commonly sealed in an inert atmosphere such as nitrogen or argon, along with a desiccant such as alumina, bauxite, calcium sulfate, clays, silica gel, zeolites, alkaline metal oxides, alkaline earth metal oxides, sulfates, or metal halides and perchlorates. Methods for encapsulation and desiccation include, but are not limited to, those described in US6,226,890. In addition, barrier layers such as SiOₓ, Teflon, and alternating inorganic/polymeric layers are known in the art for encapsulation.

Devices fabricated in accordance with embodiments of the invention may be incorporated into a wide variety of consumer products, including flat panel displays, computer monitors, televisions, billboards, lights for interior or exterior illumination and/or signalling, fully transparent displays, flexible displays, laser printers, cell phones, personal digital assistants (PDAs), laptop computers, digital cameras, camcorders, viewfinders, micro-displays, vehicles, theatre or stadium screen, or a sign. Various control mechanism may be used to control devices fabricated in accordance with the present invention, including passive matrix and active matrix.

Various features and aspects of the present invention are illustrated further in the examples that follow. While these examples are presented to show one skilled in the art how to operate within the scope of this invention, they are not to serve as a limitation on the scope of the invention where such scope is only defined in the claims. Unless otherwise indicated in the following examples and elsewhere in the specification and claims, all parts and percentages are by weight and temperatures are in degrees centigrade.

### Examples

### Preparation examples

The compounds exemplifying the present invention are compiled in the following Table 1:

| Table 1: Prepared compounds | |
|---|---|
| 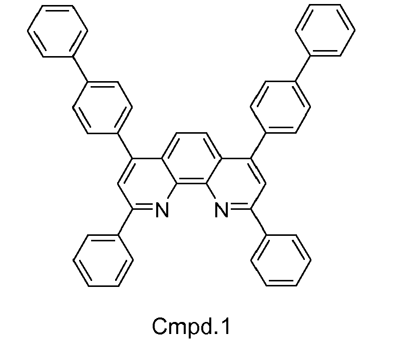 | 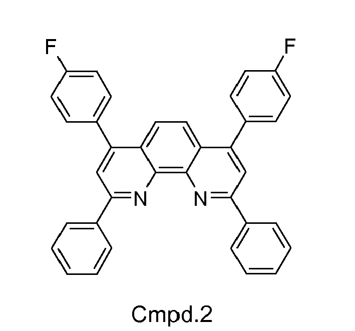 |
| 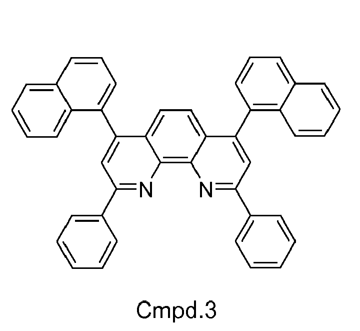 | 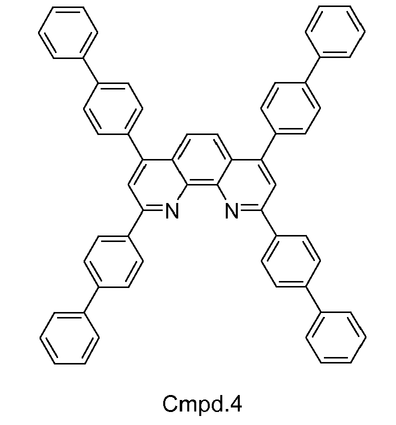 |
| 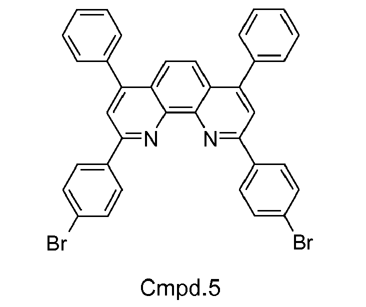 | 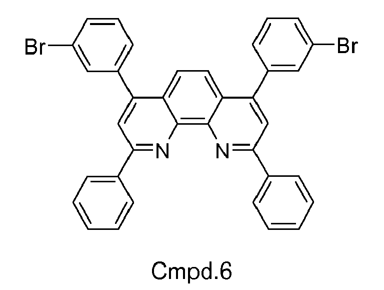 |
| 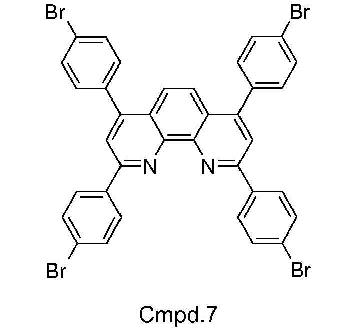 | 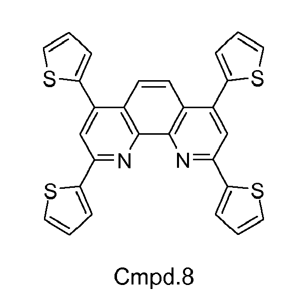 |
| 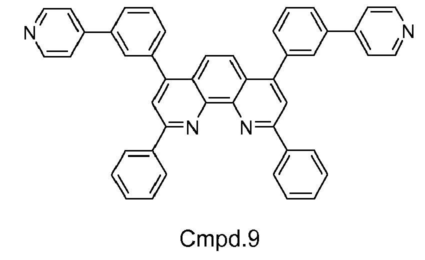 | |
| 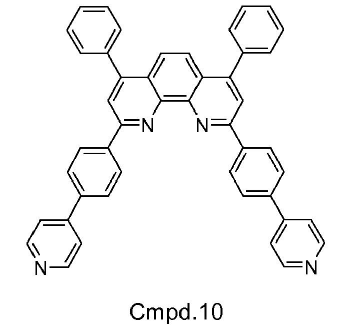 | 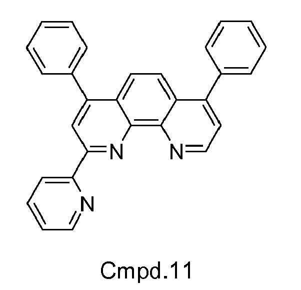 |
| 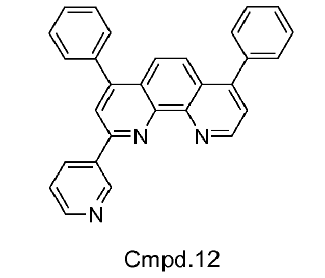 | 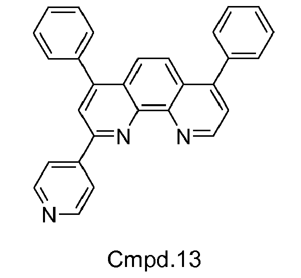 |

### Example 1 (Cmpd 1): 4,7-Bis-(biphenyl-4-yl)-2,9-diphenyl-1,10-phenanthroline

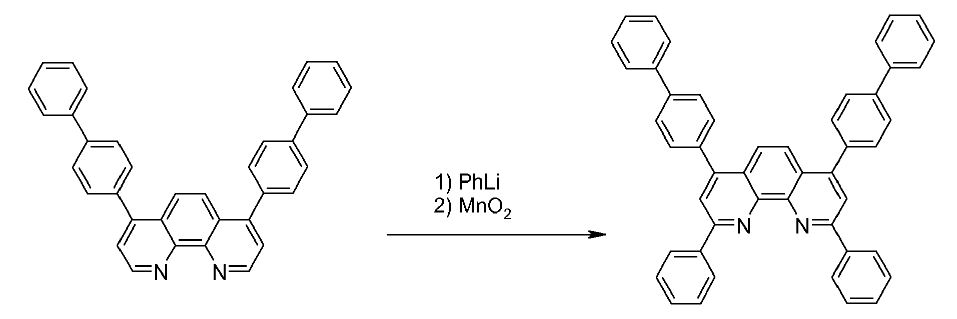

This compound is prepared from 4,7-bis-(4-biphenylyl)-1,10-phenanthroline (prepared as described in J.Org. Chem. 1641 (1962)) via addition of phenyllithium followed by oxidation with MnO₂ following the procedure described in EP 1097980/A2.
Light yellow, microcrystalline powder, mp (DSC in air, scan rate 10°Cmin⁻¹) = 318-323.9°C. ¹H-NMR (300 MHz, CF₃COOD): 8.56 (d, J=5.1 Hz, 2H), 8.47 (d, J=4.8 Hz, 2H), 8.05-6.95 (m, 28H).
MS for C₄₈H₃₂N₂ (636.80) found MH⁺ = 637.

### Example 2 (Cmpd 2): 4,7-Bis-(4-fluoro-phenyl)-2,9-diphenyl-1,10-phenanthroline

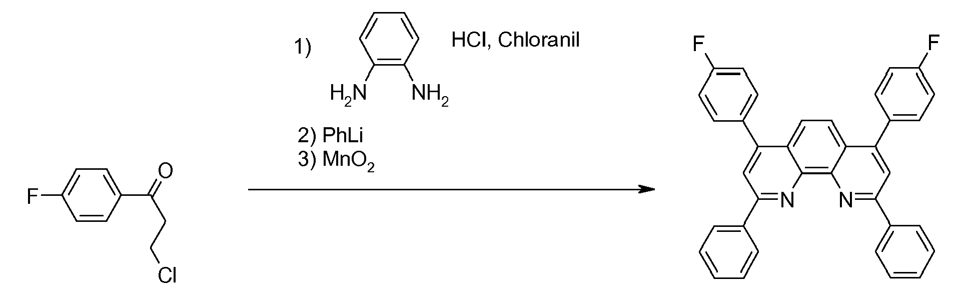

This compound is prepared via addition of phenyllithium to 4,7-di-(4-fluorophenyl)-1,10-phenanthroline followed by oxidation with MnO₂ following the procedure described in EP 1097980/A2.

The intermediate 4,7-bis-(4-fluorophenyl)-1,10-phenanthroline is prepared from 3-chloro-4'-fluoropropiophenone (prepared as described in US4864028, (1989)) and 1,2-diaminobenzene following the published procedure (CS 150747, 1,10-Phenanthroline derivatives (1973)) for preparation of 2,4,7,9-tetraphenylphenanthroline from 3-chloro-propiophenone and 1,2-diaminobenzene.
Light yellow, microcrystalline powder, mp (DSC in air, scan rate 10°Cmin⁻¹) = 293.4-297.1°C. ¹H-NMR (300 MHz, CD₃Cl): 8.51 (d, J=8.7 Hz, 4H), 8.06 (s, 2H), 7.77 (s, 2H), 7.65-7.45 (m, 10H), 7.30-7.14 (m, 4H)
MS for C₃₆H₂₂F₂N₂ (520.58) found MH⁺ = 521.

### Example 3 (Cmpd 3): 4,7-Bis-(1-naphthalen-1-yl)-2,9-diphenyl-[1,10]phenanthroline

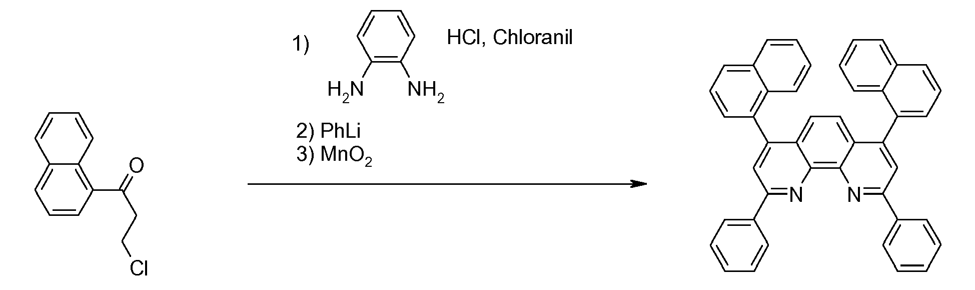

This compound is prepared via addition of phenyllithium to 4,7-di-(1-naphthyl)-1,10-phenanthroline followed by oxidation with MnO₂ following the procedure described in EP 1097980/A2.
The intermediate 4,7-Bis-(1-naphthyl)-1,10-phenanthroline is prepared from, 3-chloro-1-(1-naphthalenyl)-1-propanone (prepared as described in Tetrahedron (2001), 57(13), 2621-2634) and 1,2-diaminobenzene following the published procedure (CS 150747, 1,10-Phenanthroline derivatives (1973)) for preparation of 2,4,7,9-tetraphenylphenanthroline from 3-chloro-propiophenone and 1,2-diaminobenzene.

Light yellow, microcrystalline powder, mp (DSC in air, scan rate 10°Cmin⁻¹) = 328.7-334.3°C. ¹H-NMR (300 MHz, CD₃Cl): 8.56 (d, J= 8.4 Hz, 4H), 8.19 (s, 2H), 8.00-7.9 (m, 4H), 7.65-7.15 (m, 18H).
MS for C₄₄H₂₈N₂ (584.72) found MH⁺ = 585.

### Example 4 (Cmpd 4): 2,4,7,9-Tetrakis-biphenyl-4-yl-1,10-phenanthroline

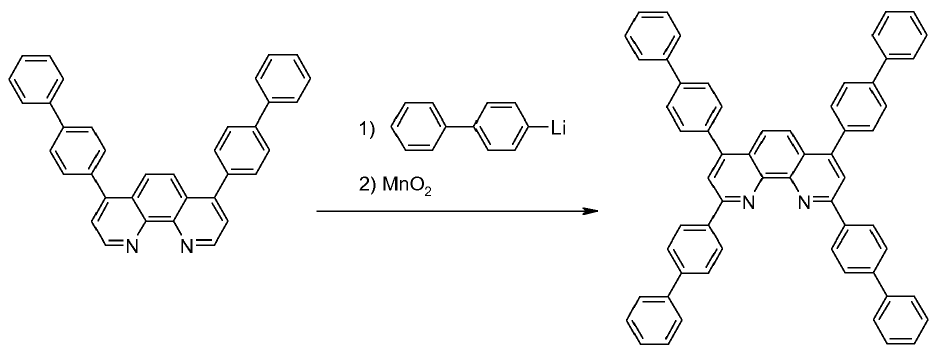

This compound is prepared from 4,7-bis-(4-biphenylyl)-1,10-phenanthroline (prepared as described in J.Org. Chem. 1641 (1962)) via addition of 1-biphenylyllithium (prepared as described in described in EP 1097980/A2) followed by oxidation with MnO₂ following the procedure described in EP 1097980/A2.
Light yellow, microcrystalline powder, mp (DSC in air, scan rate 10°Cmin⁻¹) = 388.1-391.9°C. ¹H-NMR (300 MHz, CD₃Cl): 8.66 (d, J= 8.4 Hz, 4H), 8.22 (s, 2H), 7.95-7.71 (m, 24H), 7.54-7.37 (m, 10H).
MS for C₆₀H₄₀N₂ (789.00) found M=789.

### Example 5 (Cmpd 5): 2,9-Bis-(4-bromophenyl)- 4,7-diphenyl-1,10-phenanthroline

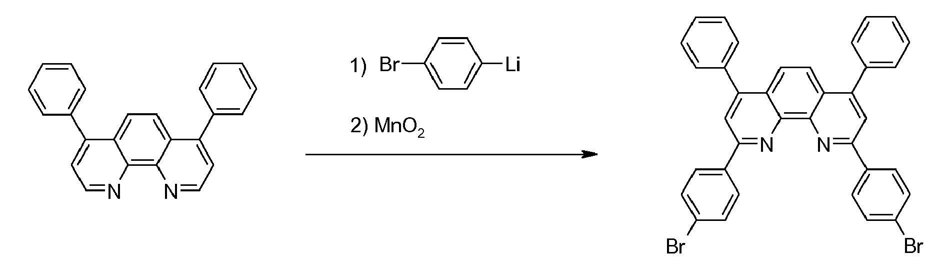

This compound is prepared from 4,7-diphenyl-1,10-phenanthroline (commercial) via addition of 4-bromophenyllithium (prepared as described in Journal of Organometallic Chemistry (1983), 251 (2), 139-48) followed by oxidation with MnO₂ following the procedure described in EP 1097980/A2.
Light yellow, microcrystalline powder, mp (heating microscope) = 323-330°C.
¹H-NMR (300 MHz, CD₃Cl): 8.38 (d, J= 8.4 Hz, 4H), 8.06 (s, 2H), 7.83 (s, 2H), 7.75-7.71 (m, 4H), 7.60-7.49 (m, 10H).
MS for C₃₆H₂₂Br₂N₂ (642.40) found M=642.

### Example 6 (Cmpd 6): 4,7-Bis-(3-bromophenyl)-2,9-diphenyl-1,10-phenanthroline

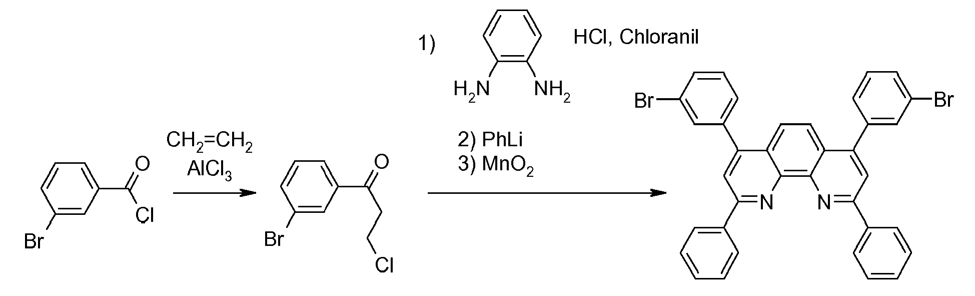

3-Bromobenzoylchloride is converted into 3-chloro-3'-bromopropiophenone using the procedure described in Bioorganic & Medicinal Chemistry Letters (2005), 15(3), 589-593 for synthesis of phenyl-2-chloroethyl ketone.
The 3-chloro-3'-bromopropiophenone is converted into the intermediate 4,7-bis-(3-bromophenyl)-1,10-phenanthroline following the published procedure (CS 150747, 1,10-Phenanthroline derivatives (1973)) for preparation of 2,4,7,9-tetraphenylphenanthroline from 3-chloro-propiophenone and 1,2-diaminobenzene.
Addition of phenyllithium followed by oxidation with MnO₂ using the procedure described in EP 1097980/A2 affords the title compound.

Light yellow, microcrystalline powder, mp (heating microscope) = 287-290°C.
¹H-NMR (300 MHz, CD₃Cl): 8.51 (d, J= 8.7 Hz, 4H), 8.08 (s, 2 H), 7.79-7.19 (m, 16H).
MS for C₃₆H₂₂Br₂N₂ (642.40) found M=642.

### Example 7 (Cmpd 7): 2,4,7,9-Tetrakis-(4-bromophenyl)-1,10-phenanthroline

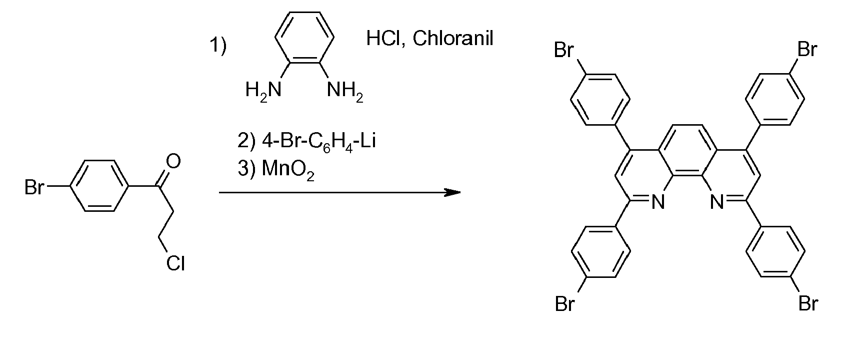

The 3-chloro-4'-bromopropiophenone (commercial) is converted into the intermediate 4,7-bis-(4-bromophenyl)-1,10-phenanthroline following the published procedure (CS 150747, 1,10-Phenanthroline derivatives (1973)) for preparation of 2,4,7,9-tetraphenylphenanthroline from 3-chloro-propiophenone and 1,2-diaminobenzene.
Addition of 4-bromophenyllithium (prepared as described in Journal of Organometallic Chemistry (1983), 251 (2), 139-48) followed by oxidation with MnO₂ using the procedure described in EP 1097980/A2 affords the title compound.
Light yellow, microcrystalline powder, mp (heating microscope) = >350°C.
¹H-NMR (300 MHz, CD₃Cl): 8.45 (d, J= 8.1 Hz, 4H), 8.04 (s, 2 H), 7.80-7.30 (m, 14H).
MS for C₃₆H₂₀Br₄N₂ (800.19) found M=800.

### Example 8 (Cmpd 8): 2,4,7,9-Tetrakis-(2-thienyl)-1,10-phenanthroline

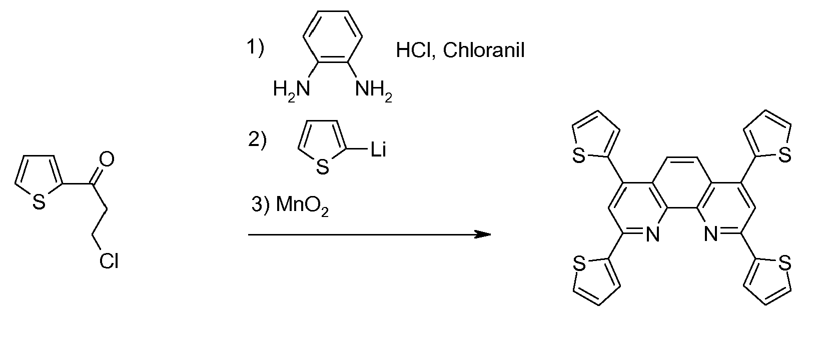

The 3-chloro-1-(2-thienyl)-1-propanone (commercial) is converted into the intermediate 4,7-bis-(2-thienyl)-1,10-phenanthroline following the published procedure (CS 150747, 1,10-Phenanthroline derivatives (1973)) for preparation of 2,4,7,9-tetraphenylphenanthroline from 3-chloro-propiophenone and 1,2-diaminobenzene.
Addition of 2-thienyllithium (commercial) followed by oxidation with MnO₂ using the procedure described in EP 1097980/A2 affords the title compound.
Light yellow, microcrystalline powder, mp (heating microscope) = ∼290°C (decomposition). ¹H-NMR (300 MHz, CD₃Cl): 8.15 (s, 2 H), 8.04 (s, 2 H), 7.89 (d, J=0.9Hz, 2H), 7.58-7.51 (m, 4H), 7.45-7.44 (m, 2H), 7.28-7.20 (m, 4H).
MS for C₂₈H₁₆N₂S₄ (508.71) found M=508.

### Example 9 (Cmpd 9): 4,7-Bis-(3-(4-pyridyl)-phenyl)-2,9-diphenyl)-1,10-phenanthroline

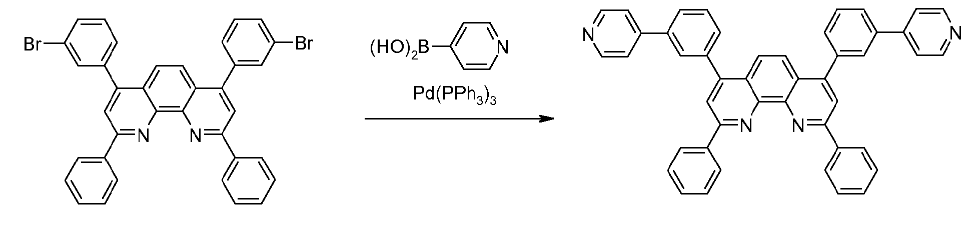

To the deoxygenated mixture of 4,7-bis-(3-bromophenyl)-2,9-diphenyl-1,10-phenanthroline (cmpd.6) (1.615 g, 0.0025 mol), pyridine-4-boronic acid (1.031 g, 0.0075 mol), potassium carbonate (3.475 g, 0.025 mol), ethanol (10 ml), water (5 ml) and toluene (25 ml) is added tetrakis-(triphenylphosphine)palladium (0.23g, 0.0002 mol). The mixture is then stirred under reflux in argon atmosphere for 18 h. It is then cooled to room temperature and diluted with dichloromethane (500 ml) and water (50 ml). The organic layer is separated, washed with water and evaporated. The residue is purified by chromatography on silicagel with dichloromethane-ethylacetate (1:1) and then dichloromethane -2-propanol (4:1). The pure fractions are crystallized from dimethylformamide to afford 1.16 g of the title compound.
Light yellow, microcrystalline powder, mp (DSC in air, scan rate 10°Cmin⁻¹) = 309.5-313.7°C. ¹H-NMR (300 MHz, CD₃Cl): 8.71 (d, J= 6.3 Hz, 4H), 8. 54 (d, J=8.7 Hz, 4H), 8.16 (s, 2H), 7.89-7.51 (m, 20H).
MS for C₄₆H₃₀N₄ (638.78) found M=638.6.

### Example 10 (Cmpd 10): 2,9-Bis-(4-(4-pyridyl)phenyl)-4,7-diphenyl-1,10-phenanthroline

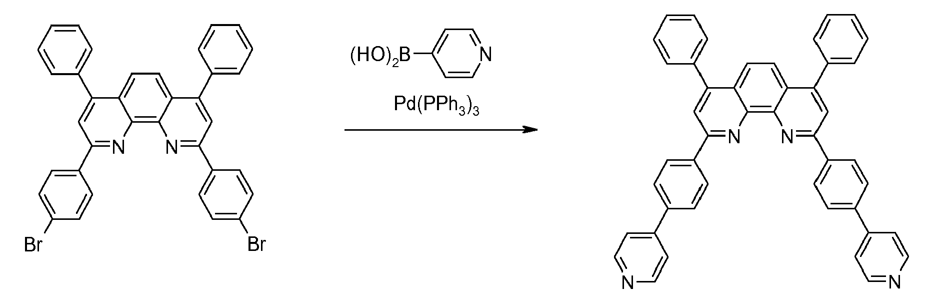

To the deoxygenated mixture of 2,9-Bis-(4-bromophenyl)- 4,7-diphenyl-1,10-phenanthroline (cmpd.5) (1.615 g, 0.0025 mol), pyridine-4-boronic acid (1.031 g, 0.0075 mol), potassium carbonate (3.475 g, 0.025 mol), ethanol (10 ml), water (5 ml) and toluene (25 ml) is added tetrakis-(triphenylphosphine)palladium (0.23g, 0.0002 mol). The mixture is then stirred under reflux in argon atmosphere for 16 h. It is then cooled to room temperature and diluted with dichloromethane (600 ml) and water (50 ml). The organic layer is separated, washed with water and diluted with ethanol (300 ml). The volume of the solution is reduced by distillation to 100 ml. The precipitated solid is filtered off and purified by chromatography on silicagel with dichloromethane-ethylacetate (1:1) and then dichloromethane -2-propanol (4:1). The pure fractions are crystallized from dimethylformamide to afford 1.2 g of the title compound. Light yellow, microcrystalline powder, mp (DSC in air, scan rate 10°Cmin⁻¹) = 355.2-359.5°C. ¹H-NMR (300 MHz, CD₃Cl): 8.75-8.68 (m, 8 H), 8. 18 (s, 2 H), 7.96-7.89 (m, 6 H), 7.79 (d, J=6.3 Hz, 4H), 7.64-7.53 (m, 10H).
MS for C₄₆H₃₀N₄ (638.78) found M=638.7.

### Example 11 (Cmpd 11): 4,7-Diphenyl-2-(2-pyridyl)-1,10-phenanthroline

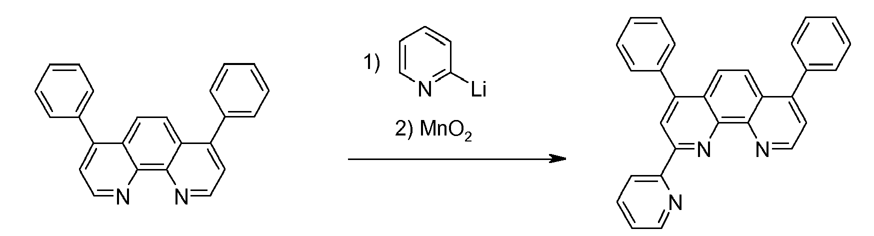

n-Butyllithium (18.8 ml of a 1.6 M solution in hexane, 0.03 mol) is added under argon to a cold (-78°C) tetrahydrofurane (100 ml). 2-Bromopyridine (4.75 g, 0.03 mol) is then added during 15 minutes while keeping the temperature at -78 °C. The brown solution is stirred 50 minutes at -78°C, thereafter finely powdered 4,7-diphenyl-1,10-phenanthroline (6.64g, 0.02 mol) is added and the mixture is allowed to warm to room temperature during 2 h. Methanol (20 ml) is then added and the brown solution is evaporated on a rotary evaporator. The residue is dissolved in dichloromethane (300 ml) and methanol (10 ml), manganese dioxide (30 g) is added and the mixture is stirred at room temperature 12 h. The solids are removed by filtration, the filtrate is evaporated and the residue is chromatographed on silica gel with dichloromethane-2-propanol (9:1). The pure fractions are crystallized from dichloromethane-ethanol to afford 2.12 g of the title compound.

Light yellow, microcrystalline powder, mp (DSC in air, scan rate 10°Cmin⁻¹) = 227.8-233.7°C. ¹H-NMR (300 MHz, CD₃Cl): 9.32 (d, J=4.8 Hz, 1H), 9.07 (d, J=7.8 Hz, 1H), 8.80 (s, 1H), 8.73 (d, J=6.3 Hz, 1H), 7.99-7.85 (m, 3H), 7.63-7.36 (m, 12H).
MS for C₂₉H₁₉N₃ (409.49) found M=409.69.

### Example 12 (Cmpd 12): 4,7-Diphenyl-2-(3-pyridyl)-1,10-phenanthroline

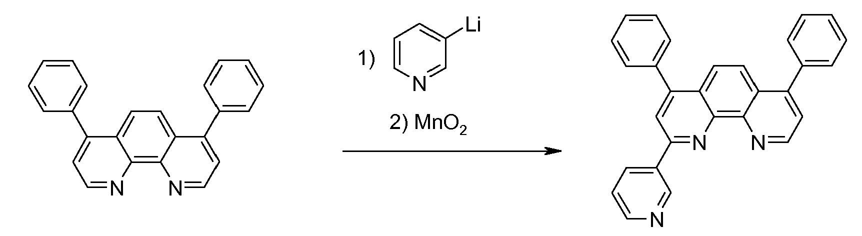

n-Butyllithium (18.8 ml of a 1.6 M solution in hexane, 0.03 mol) is added under argon to a cold (-78°C) tetrahydrofurane (100 ml). 3-bromopyridine (4.75 g, 0.03 mol) is then added during 15 minutes while keeping the temperature at -78°C. The brown solution is stirred 50 minutes at -78°C, thereafter finely powdered 4,7-diphenyl-1,10-phenanthroline (6.64g, 0.02 mol) is added and the mixture is allowed to warm to room temperature during 40 minutes. Methanol (30 ml) is then added and the brown solution is evaporated on a rotary evaporator. The residue is dissolved in dichloromethane (300 ml) and methanol (10 ml), manganese dioxide (30 g) is added and the mixture is stirred at room temperature 12 h. The solids are removed by filtration, the filtrate is evaporated and the residue is crystallized from dichloromethane-ethanol and then o-dichlorobenzene to afford 2.1 g of the title compound.

Light yellow, microcrystalline powder, mp (DSC in air, scan rate 10°Cmin⁻¹) = 268.3-273.5°C. ¹H-NMR (300 MHz, CD₃Cl): 9.46 (d, J=1.8 Hz, 1H), 9.31 (d, J=4.5 Hz, 1H), 8.86 (d, J=11.7 Hz, 1H), 8.73 (d, J=6.6 Hz, 1H), 8.08 (s, 1H), 7.89 (s, 2H), 7.64-7.48 (m, 12H).
MS for C₂₉H₁₉N₃ (409.49) found M=409.69.

### Example 13 (Cmpd 13): 4,7-Diphenyl-2-(4-pyridyl)-1,10-phenanthroline

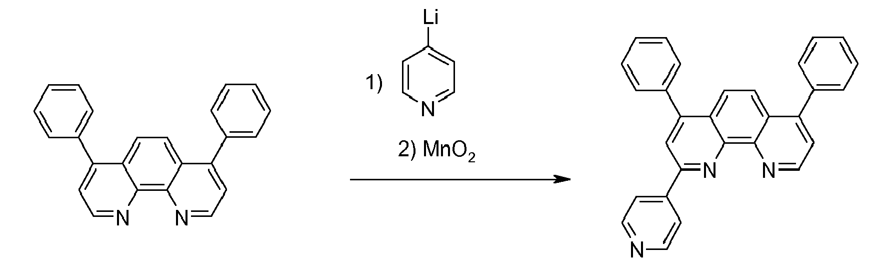

n-Butyllithium (37.6 ml of a 1.6 M solution in hexane, 0.06 mol) is added under argon to a cold (-78°C) tetrahydrofurane (50 ml). 4-Bromopyridine (9.5 g, 0.06 mol) is then added during 30 minutes while keeping the temperature at -78°C. The brown solution is stirred 50 minutes at -78°C, thereafter finely powdered 4,7-diphenyl-1,10-phenanthroline (6.64g, 0.02 mol) is added and the mixture is allowed to warm to room temperature during 2 h. Methanol (20 ml) is then added and the brown solution is evaporated on a rotary evaporator. The residue is dissolved in dichloromethane (300 ml) and methanol (20 ml), manganese dioxide (30 g) is added and the mixture is stirred at room temperature 12 h. The solids are removed by filtration, the filtrate is evaporated and the residue is chromatographed on silica gel with dichloromethane-2-propanol (5:1). The pure fractions are crystallized from dichloromethane-acetonitrile to afford 1.0 g of the title compound.

Light yellow, microcrystalline powder, mp (heating microscope ) = 195-198°C.
¹H-NMR (300 MHz, CD₃Cl): 9.33 (d, J=4.5 Hz, 1H), 8.82 (d, J=6 Hz, 2H), 8.40 (d, J=6 Hz, 2H), 8.10 (s, 1H), 7.90 (d, J=1.8 Hz, 2H), 7.65 (d, J=4.5Hz, 1H), 7.60-7.50 (m, 10H).
MS for C₂₉H₁₉N₃ (409.49) found M=409.6.

### Application examples

### QLED's devices fabrication and measurements

OLED's devices were fabricated by thermal evaporation in high vacuum (<10⁻⁶ mbar). The anode consisted of ca. 1200 A of indium tin oxide (ITO) previously deposited on a glass substrate. The cathode consisted of 10 A of LiF followed by 1000 A of Al. All devices were tested immediately after preparation, without encapsulation, in the nitrogen atmosphere of a glove box (<1 ppm of H₂O and O₂).

The organic stack consisted sequentially, from the ITO surface, of 600 A of 2-TNATA (4,4',4''-Tris(N-(naphth-2-yl)-N-phenyl-amino)triphenylamine) as the hole injection layer (HIL), 300 A of 4,4'-bis[N-(1-naphtyl)-N-phenylamino]biphenyl (aNPD) as the hole transport layer, 300 A of a aluminum(III) bis(2-methyl-8-hydroxyquinolinato)-4-phenylphenolate (BAIQ) doped with 10% of red emitter Ir(piq)2(acac) as the emissive layer. Device examples 1, 2, 3 and 4 consisted respectively of 30 nm of Cmpd.1, Cmpd.2, BCP (2,9-Dimethyl-4,7-diphenyl-1,10-phenanthroline), TPP (2,4,7,9-tetraphenyl-1,10-phenanthroline) as the electron transport layer.

The current efficiency (Eff[cd/A] at 1000 cd/m² ) along with the onset voltage (V[V] @1000 cd/m²) and maximum luminance at 14 V measured for devices 1-4 is reported in the Table 2.

| Table 2: Comparison of Compounds 1, 12 with state of the art in OLED's | | | | |
|---|---|---|---|---|
| Device # | ETM (30nm) | Eff.[cd/A] @1000cd/m² | Onset V[V] @1000 cd/m² | Max. Lum. [cd/M2] @ 14V |
| 1 | Cmpd 1 | 7.5 | 7.8 | 9200 |
| 2 | Cmpd 12 | 8.5 | 9.0 | 9500 |
| 3 | BCP | 8.3 | 9.3 | 7300 |
| 4 | TPP | 8.7 | 8.5 | 9000 |

Clearly, Cmpd.1 and Cmpd.12 show higher maximum luminance than the comparative compounds BCP and TPP. An additional advantage of Cmpd.1 is the reduced onset voltage in comparison with the state-of-the art compounds BCP and TPP.

## Claims

1. A compound of formula (I) or (II) 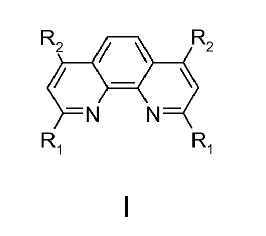 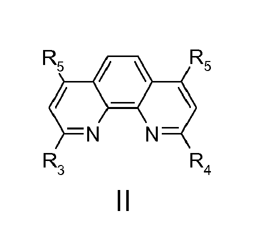 wherein
in formula (I) R₁ and R₂ are independently substituted or unsubstituted C₆-C₂₅ aryl,
with the proviso that,
if R₁ is unsubstituted phenyl, R₂ is not unsubstituted phenyl, and
if R₁ is aryl other than unsubstituted phenyl, R₂ is not unsubstituted phenyl, substituted or unsubstituted 3-perylenyl ( 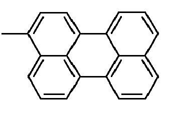 ), 2-fluorenyl ( 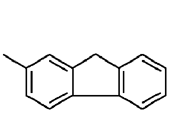 ) or 8-fluoranthenyl ( 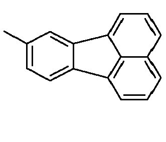 ), and
in formula (II) R₃ is C₃-C₂₅ heteroaryl, R₄ is H, C₁- C₁₈ alkyl, C₄- C₁₂ cycloalkyl, C₂-C₁₈alkenyl, C₂-C₁₈alkinyl, C₆-C₂₅ aryl, or C₃-C₂₅ heteroaryl, and R₅ is C₅- C₁₂ cycloalkyl, C₆-C₂₅ aryl, or C₃-C₂₅ heteroaryl, wherein R₃ to R₅ may optionally be substituted.

2. A compound of formula (I) or (II) according to claim 1, wherein
in formula (I) R₁ and R₂ are independently substituted or unsubstituted C₆-C₂₀ aryl, and
in formula (II) R₃ is C₃-C₁₂ heteroaryl, R₄ is H, C₆- C₁₂ alkyl, C₆-C₂₅ aryl, or C₃-C₁₂ heteroaryl, and R₅ is C₆-C₁₂ aryl or C₃-C₁₂ heteroaryl.

3. A compound of formula (I) or (II) according to claim 1 and/or 2, wherein
aryl is selected from phenyl, biphenylyl, naphthyl, fluorenyl, anthrenyl, anthracenyl, phenanthrenyl, perylenyl, and pyrenyl;
heteroaryl is selected from pyrryl, thienyl, triazolyl, pyrazolyl, oxazolyl, imidazolyl, pyridyl, pyrimidyl, pyridazyl, triazinyl, chinolyl, isochinolyl, carbazolyl, quinazolinyl, quinoxylinyl, phenazinyl, phthalazinyl, acridinyl, pteridyl, benzimidazolyl, benzofuranyl, benzoxazolyl, xanthenyl, phenoxazinyl, and phenothiazinyl; and
the optional substituents on R₁ to R₅ independently are selected from C₁- C₁₈ alkyl, C₂-C₁₈alkenyl, C₂-C₁₈alkinyl, C₄-C₁₂ cycloalkyl, halogen, cyano, C₁-C₁₈ alkoxy, -COO-C₁-C₁₈ alkyl, -CONH₂, -CO-NH-C₁-C₁₈ alkyl, -CO-N(C₁-C₁₈ alkyl)₂, -CO-NH-C₆-C₂₅ aryl, -CO-N(C₆-C₂₆ aryl)₂, -NH₂, -NH-C₁-C₁₈ alkyl, -N(C₁-C₁₈ alkyl)₂, -NH- C₆-C₂₅ aryl, -N(C₆-C₂₆ aryl)₂.

4. An electroluminescent device, comprising a compound of formula (I) or (II) as defined in any of claims 1-3.

5. An electroluminescent device according to claim 4, comprising a cathode, an anode, and there between a light emitting layer containing a host material and a phosphorescent light-emitting material, wherein the compound of formula (I) or (II) is the host material.

6. An electroluminescent device according to claim 4, containing an electron transport layer comprising the compound of formula (I) or (II).

7. An electroluminescent device according to claim 4, containing a hole blocking layer comprising the compound of formula (I) or (II).

8. Use of a compound of formula (I) or (II) according to any of claims 1-3 for solar cells..
